# EUROPEAN PATENT SPECIFICATION

(11) **EP 2 233 573 B1**
(45) Date of publication and mention of the grant of the patent: **23.10.2013**
(21) Application number: 08860108.3
(22) Date of filing: 28.11.2008
(51) Int. Cl.: C12N 15/113, C07H 21/00, A61K 48/00

(54) **A COMPLEX MOLECULE INTERFERING THE EXPRESSION OF TARGET GENES AND ITS PREPARING METHODS**
Komplexes MOLEKÜL mit Interferenz mit der EXPRESSION von Zielgenen und seine Vorbereitungsverfahren
MOLÉCULE COMPLEXE INTERFÉRANT AVEC L'EXPRESSION DE GÈNES CIBLES, ET SES MÉTHODES DE PRÉPARATION

(30) Priority: 29.11.2007 CN 200710196386
(43) Date of publication of application: 29.09.2010
(73) Proprietor: Suzhou Ribo Life Science Co., Ltd, Jiangsu 215347 (CN)
(72) Inventor: XI, Zhen, Tianjin 300071 (CN); LIANG, Zicai, Beijing 100871 (CN); CAO, Liqiang, Tianjin 300071 (CN); ZHANG, Junbin, Tianjin 300071 (CN); HUANG, Jinyu, Tianjin 300071 (CN)
(74) Representative: Lind, Urban Arvid Oskar
(86) International application number: PCT/CN2008/073241
(87) International publication number: WO 2009/074076

(56) References cited:
- EP-A1- 2 256 191
- WO-A2-03/070918
- WO-A2-2004/015075
- WO-A2-2007/125429
- CN-A- 1 675 359
- CN-A- 1 867 672
- PUNNA SREENIVAS ET AL: "Head-to-tail peptide cyclodimerization by copper-catalyzed azide-alkyne cycloaddition", ANGEWANDTE CHEMIE. INTERNATIONAL EDITION, vol. 44, no. 15, 8 April 2005 (2005-04-08) , pages 2215-2220, XP002567966, ISSN: 1433-7851
- NAKANE MASANORI ET AL: "Triazole-linked dumbbell oligodeoxynucleotides with NF-kappa B binding ability as potential decoy molecules", JOURNAL OF ORGANIC CHEMISTRY, vol. 73, no. 5, March 2008 (2008-03), pages 1842-1851, XP002616289, ISSN: 0022-3263
- ABE N ET AL: "Dumbbell-Shaped Nanocircular RNAs for RNA Interference", JOURNAL OF THE AMERICAN CHEMICAL SOCIETY, vol. 129, no. 49, 12 December 2007 (2007-12-12), pages 15108-15109, XP008120979, ISSN: 0002-7863

## Description

### Field of the invention

The present invention relates to a complex molecule interfering the expression of target genes and the methods for preparing the complex molecule.

### Background of the invention

Discovered in recent years, the small interfering RNA (RNAi, or siRNA) is a type of small RNA that interferes the expression of genes. The main mechanism of siRNA is that it interferes the expression of genes via complement of its antisense RNA and mRNA of target genes. Due to the fact that siRNA specially inhibits the expression of target genes, its application prospect in the field of pharmaceutical is promising. However, because the exogenous siRNA has poor chemical stability, short remaining time in blood, and poor cell and tissue penetration, these disadvantagesseverely hinder the application of exogenous siRNA to inhibit the expression of target genes.

The poor chemical stability of siRNA is not caused by the degradation of its double-stranded form, but it is due to the fact that its single strand is degraded by RNase during hybridization-unwinding balance of double strands-single strand (which is also known as "breath" of double-stranded nucleic acid), the balance is broken rapidly, and as a result, double-stranded siRNA unwinds and degrades rapidly. According to the above mechanism, a great deal of researches have been carried out to prohibit double strands unwinding via modification of siRNA, and improve the chemical stability and remaining time in blood. However, reported results indicate that current modification has a limited influence on the improvement of siRNA stability. For instance WO 2004/015075 disclosed "An interfering hairpin RNA having the structure X₁-L-X₂, wherein X₁ and X₂ are nucleotide sequences having sufficient complementarities to one another to form a double-stranded stem hybrid and L is a loop region comprising a non-nucleotide linker molecule, wherein at least a portion of one of the nucleotide sequences located within the double-stranded stem is complementary to a sequence of said target RNA", that is one end of both siRNA strands is linked through a non-nucleic acid molecule to form a hairpin siRNA, wherein the non-nuclei acid is selected from "polyethers, polyamines, polyesters, polyphosphodiesters, alkylenes, attachments, bioconjugates, chromophores, reporter groups, dye labeled RNAs, and non-naturally occurring nucleotide analogues or combinations thereof", however, the stability and remaining time in blood of said hairpin structure are not attractive. Moreover, functional molecules (for instance cell target recognizing molecules, lipid molecules capable of improving the cell penetration or fluorescent marker molecules) are not easy to be introduced to said comb-shape structure.

WO 03/070918 disclosed synthetic chemically modified small nucleic acid molecules, e.g. including hairpins and dumbbell-shaped structures with non-nucleotidic linkers, capable of mediating RNA interference against target nucleic acid sequences. The use of chemically modified siNA was proposed to improve various properties of native siRNA molecules through increased resistance to nuclease degradation in vivo and/or improved cellular uptake.

### Summary of the invention

The present invention aims at overcoming disadvantages of the prior siRNA, such as the poor chemical stability and short remaining time in blood, and providing a complex molecule (zipped interfering RNA, ziRNA) interfering the expression of target genes and the methods for preparing the complex molecule

The present invention provides a complex molecule interfering the expression of target genes, wherein the complex molecule contains two siRNA strands X₁ and X₂ having at least 80% complementarity, the 5' end of X₁ and 3' end of X₂ are linked through a non-nucleic acid molecule L₁, and the 5' end of X₂ and 3' end of X₁ are linked through a non-nucleic acid molecule L₂, according to Claim 1.

The present invention also provides a method for the preparation of the complex molecule interfering the expression of target genes of Claim 1, wherein it includes: preparing two modified siRNA strands having at least 80% complementarity, both 5' and 3' ends of the two modified siRNA strands having a linkable group; linking the linkable groups of 5' and 3' ends of one modified siRNA strand to the linkable groups of 3' and 5' ends of the other modified siRNA strand, respectively, according to Claim 7.

Since both 5' and 3' ends of two siRNA strands X₁ and X₂ of the complex molecule according to the present invention are linked through non-nucleic acid molecules, it is not easy to unwind and degraded for the siRNA strands, and therefore the chemical stability of siRNA and the remaining time in the blood are greatly improved. After being administered, the Dicer enzyme in the cells is utilized to release the locked siRNAs from the complex molecules, and after unwinding, the antisense strand of siRNA is released from the double-stranded siRNA to inhibit the expression of the target genes.

### Brief description of the accompanying drawings

Figure 1 illustrates the complex molecule according to the present invention;
Figure 2 illustrates results of pharmacokinetics analysis on ziRNA prepared from Example 1.
Figure 3 illustrates electrophoresis showing the stability of ziRNA prepared from Example 1.
Figure 4 illustrates electrophoresis showing the stability of general siRNA.

### Preferred embodiments

The complex molecule interfering the expression of target genes according to the present invention contains two siRNA strands X₁ and X₂ having at least 80% complementarity, the 5' end of X₁ and 3' end of X₂ are linked through a non-nucleic acid molecule L₁, and the 5' end of X₂ and 3' end of X₁ are linked through a non-nucleic acid molecule L₂. It should be noted that, the 5' end and 3' end are only used as an indication of the direction of non-nucleic acid strand, and are not limited to 5' position and 3' position, for instance 3' end could be linked through hydroxyl group on 3' position, as well as hydroxyl group on 2' or 1' position.

X₁ and X₂ of siRNA strands could be any siRNA strands interfering the expression of target genes, as long as two siRNA strands X₁ and X₂ are at least 80% complementary. Preferably, at least 90% of bases of siRNA strand X₁ or X₂ are complementary to the target genes, and more preferably, 100% of bases of siRNA strand X₁ or X₂ are complementary to the target genes. siRNA strand X₁ or X₂ could each contain 19~50 bases, preferably 19~40 bases, more preferably 19~30 bases.

Non-nucleic acid molecule L₁ is covalently linked to phosphate group or hydroxyl group of 5' end of X₁ and to phosphate group or hydroxyl group of 3' end of X₂, and non-nucleic acid molecule L₂ is covalently linked to phosphate group or hydroxyl group of 5' end of X₂ and to phosphate group or hydroxyl group of 3' end of X₁. The non-nucleic acid molecule L₁ and non-nucleic acid molecule L₂ could be any linkable group, and preferably the non-nucleic acid molecule L₁ and non-nucleic acid molecule L₂ are independently oligopeptide, polyester, polyether, alkane, alkene, alkyne and synthetic nucleic acid analog containing carboxyl group, amino group or mercapto group. The total atomic number of said non-nucleic acid molecule L₁ and non-nucleic acid molecule L₂ may be 10~100.

Said non-nucleic acid molecule L₁ and non-nucleic acid molecule L₂ are independently represented by Formula I. Also a non-nucleic acid molecule L₁ and a non-nucleic acid molecule L₂ independently represented by Formula II or Formula III are disclosed.

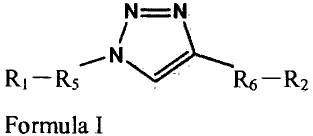

R₃-R₇-S-S-R₈-R₄ Formula II

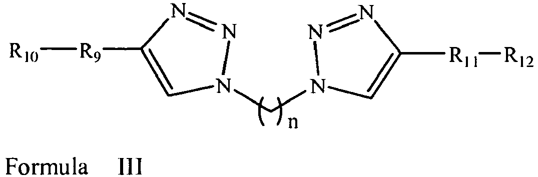

the groups R₁, R₂, R₃, R₄, R₁₀ and R₁₂ are independently carboxyl group, amino group or mercapto group;
R₅ is 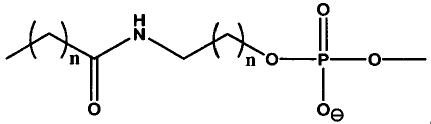 or 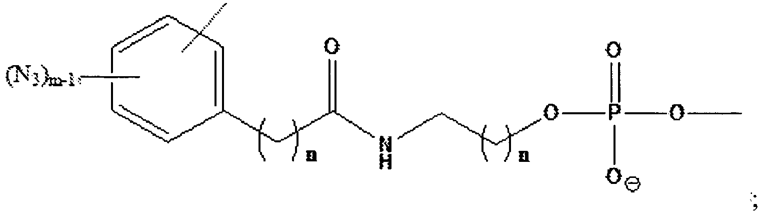 R₇ and R₈ are independently -(CH₂)ₙ-;
R₆, R₉ and R₁₁ are independently one of the following groups:

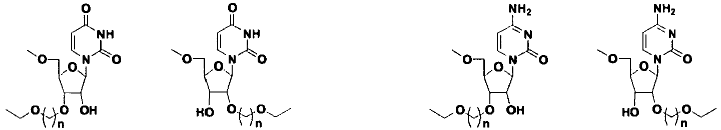

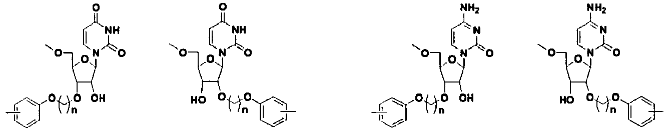

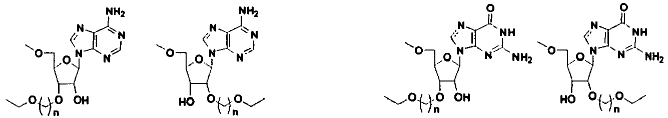

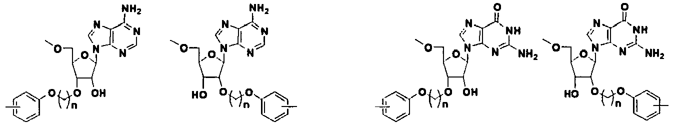

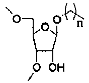

In the groups, n is independently an integer between 0~10, and m is independently an integer between 1~5.

The non-nucleic acid molecule L₁ and/or non-nucleic acid molecule L₂ could also be linked with one or more selected from cell target recognizing molecule, lipid molecule capable of improving the cell penetration, and fluorescent marker molecule; at least one of the non-nucleic acid molecule L₁ and non-nucleic acid molecule L₂ is represented by Formula I, and R₅ is 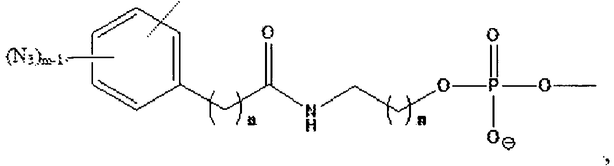 m is an integer between 2∼5, one or more selected from the cell target recognizing molecule, lipid molecule capable of improving the cell penetration, and fluorescent marker molecule is linked to azide group N₃ of R₅.

Preferably, the chemical structure of the cell target recognizing molecule, lipid molecule capable of improving the cell penetration or fluorescent marker molecules is 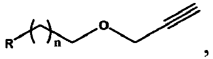 wherein R is one or more of cell target recognizing group, lipid group capable of improving the cell penetration or fluorescent marker group, and the alkynyl group in the chemical structure is linked to L₁ and/or L₂.

The alkynyl group is linked to azide group N₃ of R₅ of L₁ and/or L₂, so as to form

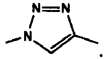

The cell target recognizing group, liquid group capable of improving the cell penetration and fluorescent marker group could be any general group having the corresponding functions.

Examples of complex molecules of present invention include but are not limited to those represented by Formula (I), Formula (II), Formula (III) and Formula (IV):

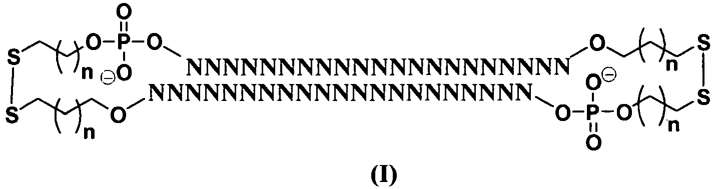

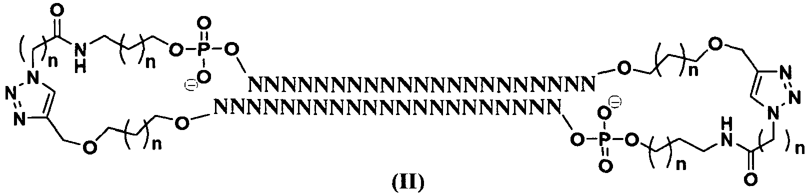

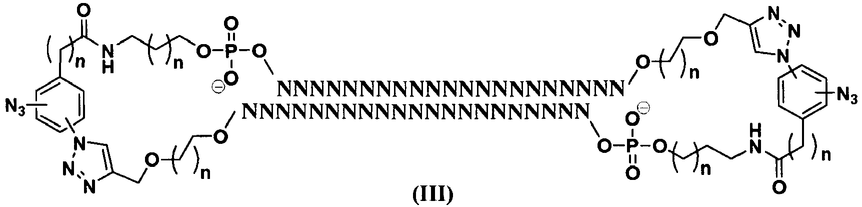

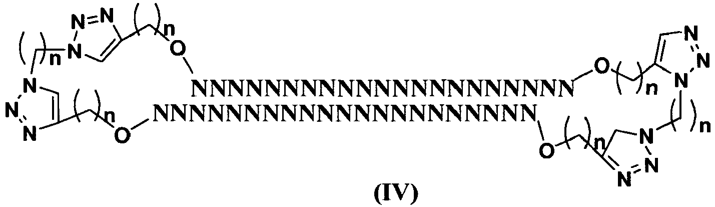

Wherein in Formula I, Formula II, Formula III and Formula IV, n may be an integer between 0~10. It should be understood that, in Formula I, Formula II, Formula III and Formula IV, strands containing N illustrate siRNA or the complementary strand of siRNA based on target genes, but the number of N does not indicate the length of siRNA.

The method for the preparation of the complex molecule interfering the expression of target genes according to the present invention includes: preparing two modified siRNA strands having at least 80% complementarity, both 5' and 3' ends of the two modified siRNA strands having a linkable group; linking the linkable groups of 5' and 3' ends of one modified siRNA strand to the linkable groups of 3' and 5' ends of the other modified siRNA strand, respectively.

According to a first embodiment, the preparing method of complex molecule includes: fixing a3 and a3' respectively, synthesizing X₁ on a3 from 3' end to 5' end, and synthesizing X₂ on a3' from 3' end to 5' end, such that a3-X₁ and a3'-X₂ are obtained; introducing a1 and a1' to 5' end of a3-X₁ and a3'-X₂, respectively, to form a1-X₁-a3 and a1'-X₂-a3'; introducing a2 and a2' to a1-X₁-a3 and a1' -X₂-a3', respectively, to form a2-a1-X₁-a3 and a2'-a1'-X₂-a3'; and then, annealing a2-a1-X₁-a3 and a2'-a1'-X₂-a3', subjecting a2 of a2-a1-X₁-a3 and a3' of a2'-a1'-X₂-a3' to a linking reaction and subjecting a3 of a2-a1-X₁-a3 and a2' of a2'-a1'-X₂-a3' to a linking reaction, such that the complex molecule is formed, wherein a1 and a1' independently are 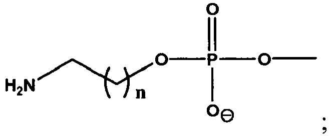 a2 and a2' independently are 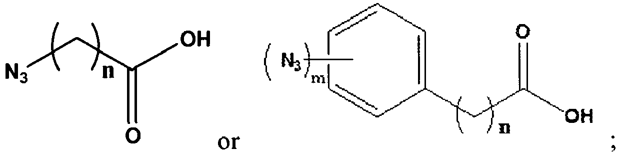 a2-a1 and a2'-a1' independently are 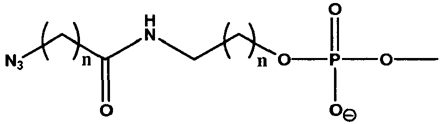 or 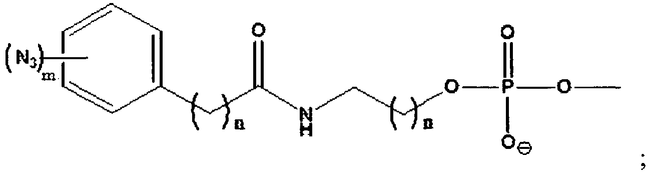 a3 and a3' are independently one of the following groups: 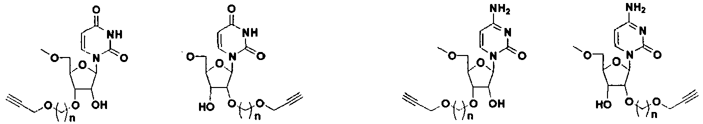 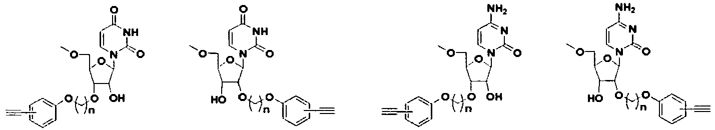 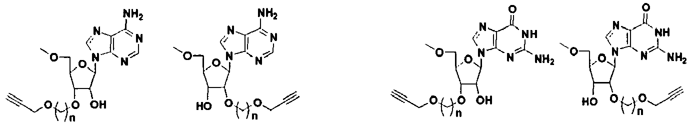 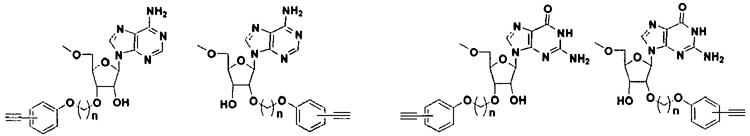 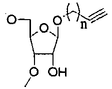 and in the groups, n is independently an integer between 0∼10, and m is independently an integer between 1~5.

For easy illustration, the a1, a1', a2, a2', a3 and a3' could be molecular states and group states, for instance, if the group state of a3 or a3' is 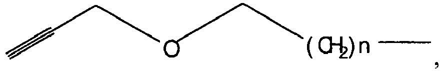 then its corresponding molecular state before reaction could be general reaction molecule containing said group, for instance it could be molecule wherein the group is linked with halogen, for instance it could be 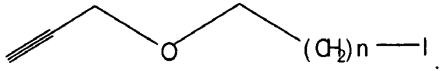 Similar examples will not be listed hereafter. a3 and a3' could be fixed via conventional method for the preparation of nucleic acid, subsequently X₁ is synthesized on a3 from 3' to 5', X₂ is synthesized on a3' from 3' to 5', and as a result, a3-X₁ and a3'-X₂ are obtained. For example, solid-phase synthesis disclosed in "Biological Chemistry" (3rd Edition, Volume I, Wang Jingyan etc. Higher Education Press, China) Page 520~521 can be used as reference. During synthesis, once desired length is obtained, protecting groups on bases could be removed by the following method including:
(1) Concentrated ammonia solution containing 0.5M LiCl (the concentration of ammonia is 25%) is used, the molecule is incubated at 55°C for 3h, and then the remaining ammonia is evaporated. And
(2) 1ml THF solution containing 1M TBAF (tetra-n-butylammonium fluoride) is added to evaporated RNA sample, it is sealed and shaken at 60°C for 24h. After quenched with 1mmol trimethylisopropoxysilane, an equal volume of DEPC (diethyl pyrocarbonate) is added to said solution to treat ddH₂O, 3M NaOAc buffer (pH=7.0) having a volume of 1/10 of mixture volume is added, and absolute ethanol having a volume of 5 times volume is added to perform precipitation.

a1 and a1' are introduced to 5' end of a3-X₁ and a3-X₂ with the aid of phosphate group, as a result, a1-X₁-a3 and a1'-X₂-a3' are formed.

a2 and a2' are introduced to a1-X₁-a3 and a1'-X₂-a3' according to Reaction Scheme (1), as a result, a2-a1-X₁-a3 and a2'-a1'-X₂-a3' are formed:

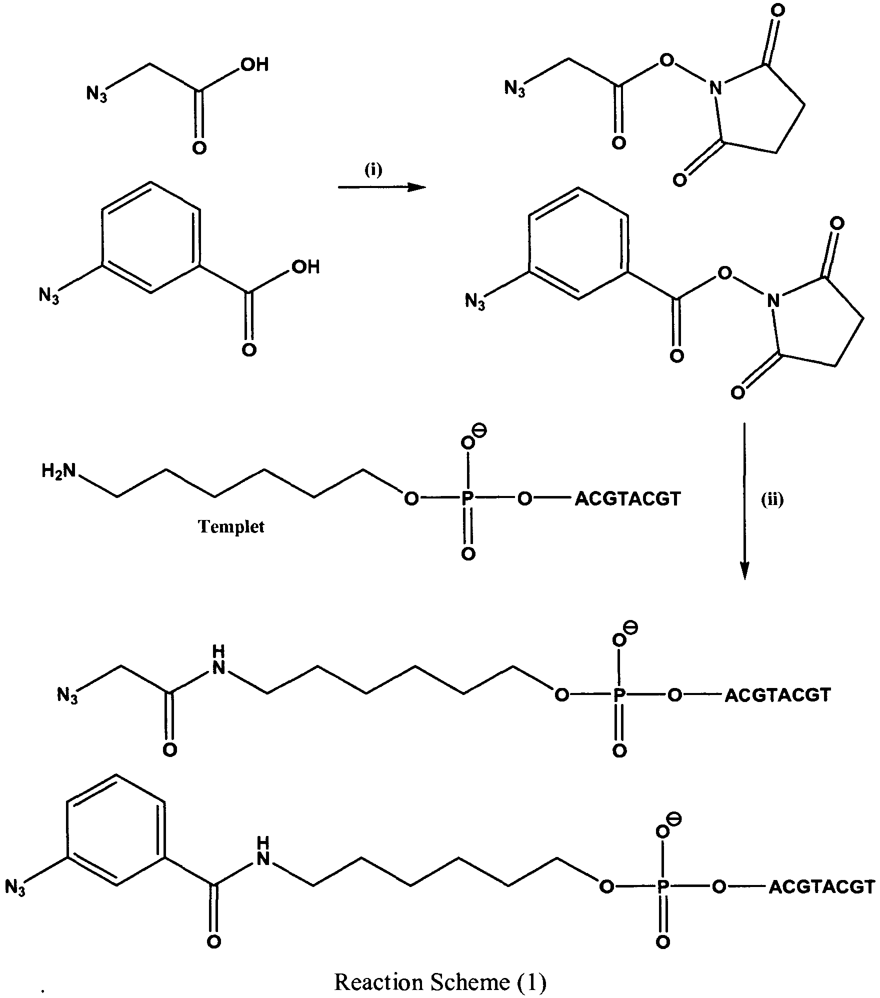

a2-a1-X₁-a3 and a2'-a1'-X₂-a3' may be annealed by conventional annealing method under conventional conditions.

The linking reaction refers to polymerization of azide group and alkynyl group, and example of polymerization of azide group and alkynyl group is shown in Reaction Scheme (2):

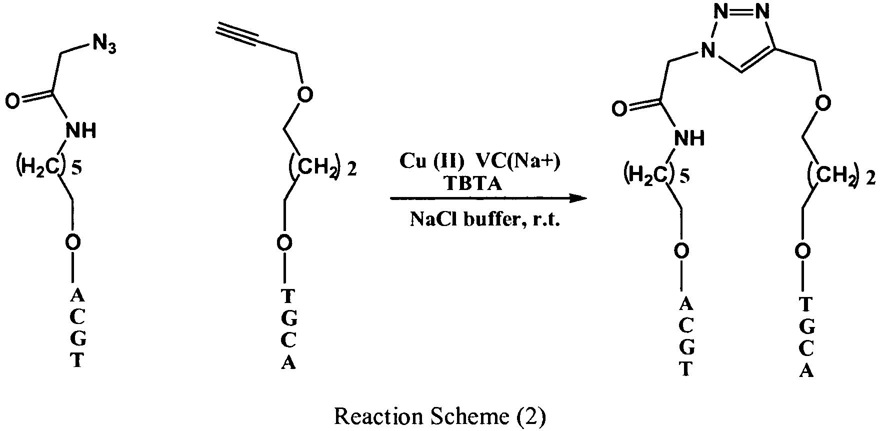

Reaction Scheme (2) only illustrates linkage of one end of X₁ and one end of X₂, and the linkage of another end can be done in the same manner.
a2 and/or a2' is preferred to be 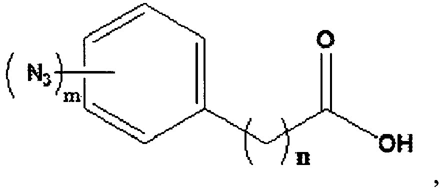 wherein m is preferred to be an integer between 2~5. According to this preferred embodiment, after a2 of a2-a1-X₁-a3 is linked to a3' of a2'-a1'-X₂-a3', and a3 of a2-a1-X₁-a3 is linked to a2' of a2'-a1'-X₂-a3', still some azide groups remain on a2 and/or a2', and the remaining azide groups may be linked with a functional molecule. The functional molecule has a group that can react with the azide group, as a result, functional molecules are linked to non-nucleic acid. Preferably, the chemical structure of said cell target recognizing molecule, lipid molecule capable of improving the cell penetration and fluorescent marker molecule is 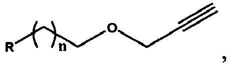 wherein R is one or more of cell target recognizing group, lipid group capable of improving the cell penetration and fluorescence marker group. The cell target recognizing group, lipid group capable of improving the cell penetration and fluorescent marker group may be conventional functional group. The alkynyl group of said functional molecule can be polymerized with the remaining azide groups of a2 and/or a2', as a result, one or more of cell target recognizing group, lipid group capable of improving the cell penetration and fluorescent marker group is introduced to said complex molecule.

According to a second embodiment, wherein the preparing method of complex molecule includes: fixing a4 and a4' respectively, synthesizing X₁ on a4 from 3' end to 5' end, and synthesizing X₂ on a4' from 3' end to 5' end, such that a4-X₁ and a4'-X₂ are obtained; introducing a5 and a5' to 5' end of a4-X₁ and a4'-X₂, respectively, to form a5-X₁-a4 and a5'-X₂-a4'; first linking a6 to one strand of a5-X₁-a4 and a5'-X₂-a4', annealing the two strands, and then linking a6 to the other strand, such that the complex molecule is formed.

Wherein a4, a4', a5 and a5' are independently one of the following groups:

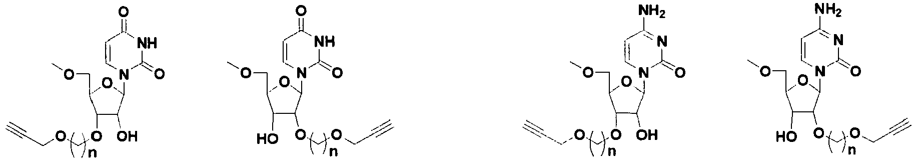

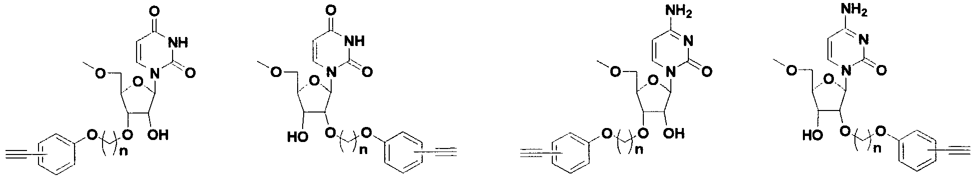

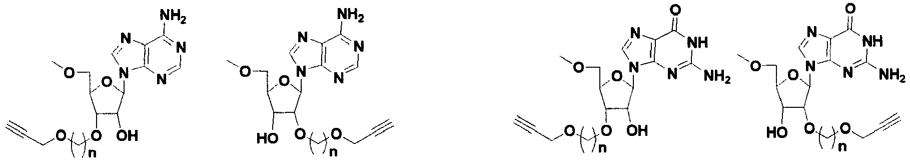

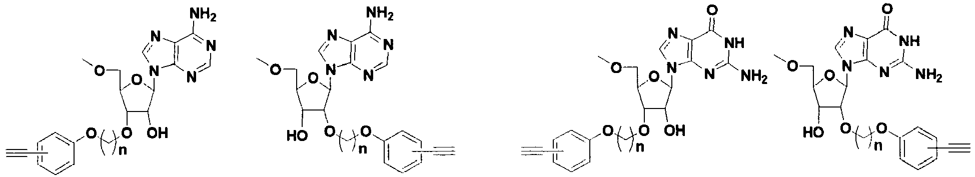

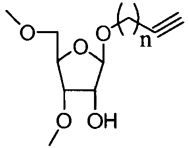

Wherein n of said groups is each an integer between 0~10.
a6 is N₃-(CH₂)ₚ-N₃,

Wherein p is an integer between 2~12.

In the second embodiment, a4 and a4' are fixed, X₁ is synthesized on a4 from 3' to 5', X₂ is synthesized on a4' from 3' to 5', as a result, a4-X₁ and a4'-X₂ are obtained, the preparation is as similar as that described in the first embodiment.

Two azide groups of a6 may react with linkable groups respectively (similar to the Reaction Scheme (2)), the reaction is also known as Click Reaction, the reaction conditions of Click Reaction could be general Click reaction conditions in the art.

a6 may be obtained via reaction of dihalohydrocarbon (containing at least 2 carbon atoms, and two halogen atoms are linked to carbon atoms at the two ends of hydrocarbon chain) with sodium azide. The synthesis of 1,2-diazidoethane, 1,3-diazidopropane and 1,4-diazidobutane is taken as samples to illustrate the synthesis of diazido-compounds:
**(1) Synthesis of 1,2-diazidoethane** 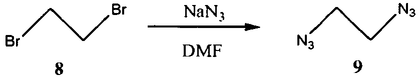
   1.87g (1mmol) compound 8, 2.6g (4mmol) sodium azide and 20ml DMF were added to a 50ml flask. The mixture was heated to 60 °C for 24h, and TLC was performed to monitor the completion of reaction. After the reaction was completed, 15ml water was added to the flask, the reaction solution was extracted with dichloromethane (20ml x 3) for 3 times; the organic phase was combined and washed with saturated NaCl solution. The solvent was evaporated, flash column chromatography was carried out, and 1.02g yellow oily compound 9 was obtained. The yield was 92%. ¹H NMR (400M, CDCl₃) δ ppm 3.41 (4H, s, CH₂×2).
**(2) Synthesis of 1,3-diazidopropane** 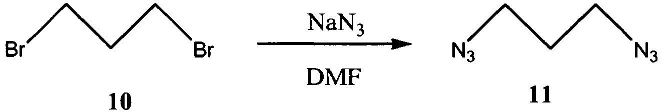
   2.00g (1mmol) compound 10, 2.6g (4mmol) sodium azide and 20ml DMF were added to a 50ml flask. The mixture was heated to 60°C for 24h, and TLC was performed to monitor the completion of reaction. After the reaction was completed, 15ml water was added to the flask, the reaction solution was extracted with dichloromethane (20ml x 3) for 3 times; the organic phase was combined and washed with saturated NaCl solution. The solvent was evaporated, flash column chromatography was carried out, and 1.08g yellow oily compound 11 was obtained. The yield was 86%. ¹H NMR(400M, CDCl₃) δ ppm 3.44-3.40 (4H, t, CH₂-N₃×2), 1.87-1.80 (2H, quintet, CH₂).
**(3) Synthesis of 1,4-diazidobutane** 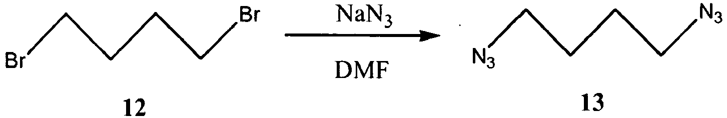
   2.13g (1mmol) compound 12, 2.6g (4mmol) sodium azide and 20ml DMF were added to a 50ml flask. The mixture was heated to 60°C for 24h, and TLC was performed to monitor the completion of reaction. After the reaction was completed, 15ml water was added to the flask, the reaction solution was extracted with dichloromethane (20ml x 3) for 3 times; the organic phase was combined and washed with saturated NaCl solution. The solvent was evaporated, flash column chromatography was carried out, and 1.13g yellow oily compound 13 was obtained. The yield was 81%. ¹H NMR (400M, CDCl₃) δ ppm 3.34 (4H, s, CH₂-N₃×2), 1.69 (4H, s, CH₂×2).

The present invention is further illustrated by the following examples.

Source of part of raw materials used in examples:
2-deoxy-D-ribose was purchased from Shanghai Haiqu Chemicals Co., Ltd. (China). Propargyl alcohol was purchased from Fluka Company.
DMTr-Cl and natural nucleoside phosphoramidite protecting monomer were purchased from Shanghai GenePharma Co., Ltd. (China).
Universal CPG was purchased from Beijing SBS Genetech Co.,Ltd. (China).
TLC was home-made thin laminate, and GF245 silicon, analytical reagent, was produced by Qingdao Haiyang Chemical Co., Ltd. (China).
Silicon for flash column chromatography, ZCH-X, macroporous, 200-300 mesh, was produced by Qingdao Haiyang Chemical Co., Ltd. (China).
Solvents for synthesis, such as pyridine, petroleum ether, ethyl acetate were purchased from Tianjin No. 6 Reagent Factory. (China).

### Example 1

The present example illustrates preparing method of complex molecule interfering the expression of target genes.

The complex molecule interfering the expression of target genes is prepared via the following steps:
1. Choosing target genes, and determining sequence of X₁ and X₂
   GAPDH (Genbank Accession No. NC_000012) was chosen as target gene to design siRNA, and its corresponding position to NC_000012 was 2700-2718bp.
   Wherein X₁ was sense strand, its sequence was: 5' GUA UGA CAA CAG CCU CAA GTT 3'; X₂ is anti-sense strand, its sequence was: 5' CUU GAG GCU GUUGUC AUA CTT 3'.
2. Preparation of nucleotide containing alkynyl group (taken base U as an example):
   (1) Based on method of the Reaction Scheme (3), 991mg protected nucleotide U (a, 1.5mmol) and 15ml absolute THF were added to a 20ml microwave reaction flask, and colorless transparent solution was obtained. Subsequently, 714mg (3mmol) raw material b containing alkynyl group, 456mg (3mmol) 1,8-diazabicyclo [5,4,0] undecene were added, and the microwave reaction flask was heated by microwave to 60°C for 1h. The reaction solution was extracted with ethyl acetate, and the organic phase was combined, washed with saturated NaCl solution, and then dried with absolute sodium sulfate. The solvent was evaporated, column chromatography was performed, and eluent was petroleum ether/ethyl acetate = 2:1. The obtained products were white solid powder c, the yield was 28%, and light yellow viscous solid, the yield was 20%. 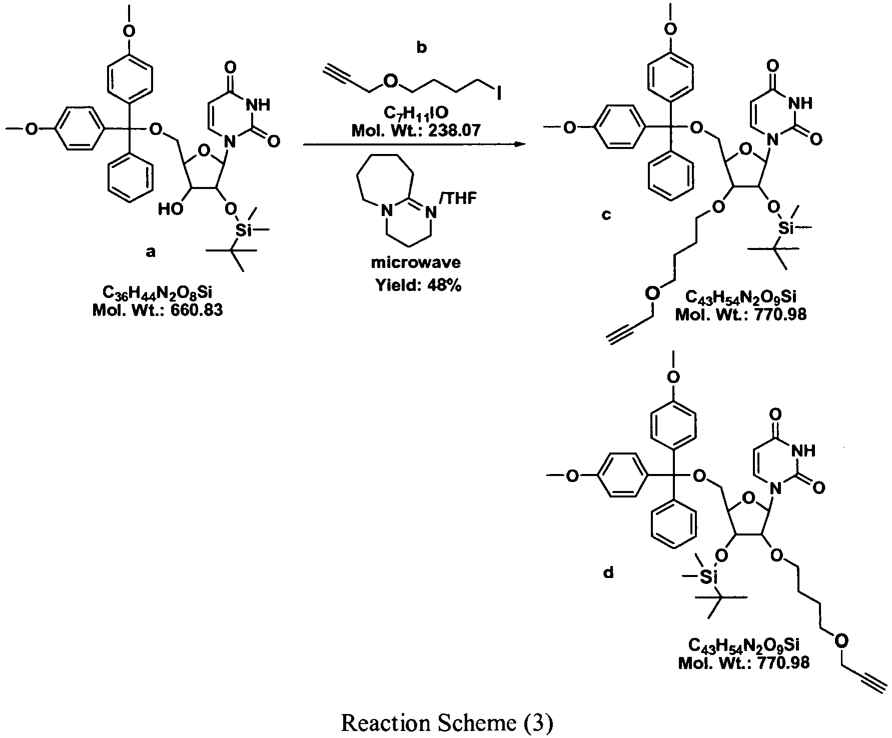
   (2) Based on method of Reaction Scheme (4), 185mg (0.24mmol) alkylating raw material d and 10ml absolute THF were added to a 25ml round-bottle flask, colorless transparent solution was obtained. 204µl (0.20mmol, 1M THF solution) was added at room temperature (16°C), the reaction mixture was mixed at room temperature for 2h, subsequently, 10ml H₂O was added to quench the reaction, THF was evaporated, the reaction solution was extracted with ethyl acetate, the organic phase was combined, washed with saturated NaCl solution, and then dried with absolute sodium sulfate. The solvent was evaporated, column chromatography was performed, and eluent was dichloromethane/ethyl acetate/methanol = 1:1:0.04. The obtained product was light yellow viscous solid, and the yield was 95%. 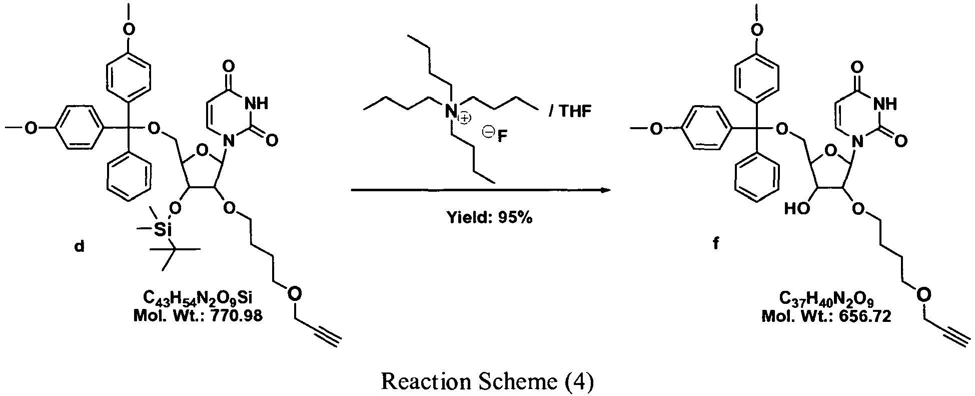
   (3) Based on method of Reaction Scheme (5), 128mg (1eq, 1mmol) raw material f containing alkynyl group and 5ml absolute dichloromethane were added to a 50ml double-neck flask under N₂ atmosphere, once raw material f was dissolved in absolute dichloromethane, 5ml absolute dichloromethane solution containing 101mg (1eq, 1mmol) diisopropylamine and 70mg (1eq, 1mmol) tetrazole was dropped thereto at room temperature (23°C). The reaction mixture was mixed, and then 5ml absolute dichloromethane solution containing 362mg (1.2eq, 1.2mmol) phosphoramidite g was added, the reaction mixture was mixed at room temperature for 2h, TLC was performed to monitor the end of reaction. Saturated sodium bicarbonate was added to quench the reaction, organic phase was separated, washed with saturated NaCl solution, and was evaporated (the operation should be carried out as fast as possible, an ice bath was applied to ensure the temperature of organic phase, and then the organic phase was vacuum evaporated, the reaction flask was first evaporated in air until the minimum pressure was reached, and then it was placed in water). Column chromatography was performed using 100~200 mesh silicon, the obtained product was yellow viscous solid, and the yield was 90%. 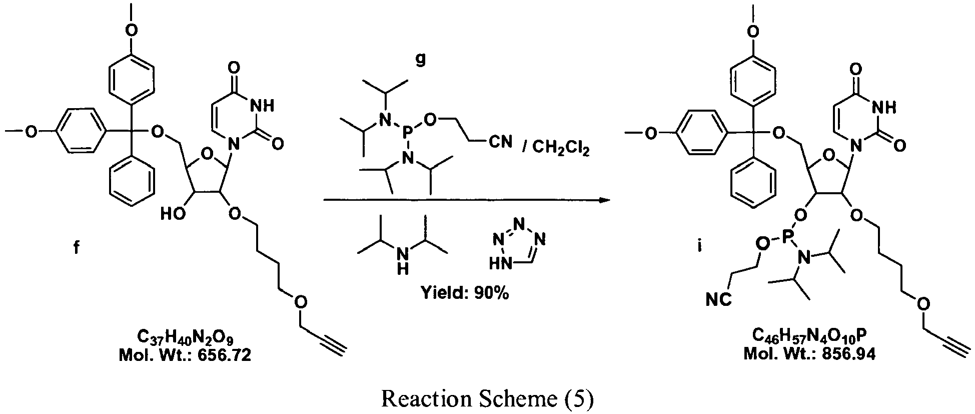
3. Reaction was proceeded according to Reaction Scheme (6):
   (i) Compound a (0.46g, 3.6mmol) and N-hydroxysuccinimide c (0.49g, 4.3mmol) were added to 20ml dichloromethane and mixed at room temperature. Subsequently, N,N'-dicyclohexylcarbodiimide b (0.89g, 4.3mmol) was added, the reaction mixture was mixed at room temperature for 4h, and TLC was performed to monitor the end of reaction. 20ml saturated NaCl solution was added to quench the reaction, the organic phase was separated, washed with water and dried with absolute sodium sulfate, and then it was vacuum evaporated. Column chromatography was performed (99: 1, dichloromethane: methanol) and colorless oily product d was obtained (0.51g, 63%).
      Obtained analytical results: IR (thin film) v: 2091, 1780, 1731cm⁻¹. δH (300MHz, CDCl₃) 3.37 (2H, t, *J*=6.6Hz, N₃CH₂), 2.77 (4H, m, COCH₂CH₂CO), 2.66 (2H, t, *J*) 7.0Hz, COCH₂-), 1.94 (2H, m, CH₂); δC (75MHz, CDCl₃) 168.9 (CO), 167.9 (CO), 50.0 (N₃CH₂), 28.1 (COCH₂-), 25.6 (COCH₂CH₂CO), 24.1 (CH₂), *m*/*z* LRMS [ES⁺, MeCN] 249 (M + Na⁺, 100%). HRMS (M + Na⁺) (C₈H₁₀N₄NaO₄): calcd, 249.0594; found, 249.0590.
   (ii) Modified RNA e containing 5' end amino-group strand was synthesized with synthesizer, and compound containing amino group and natural nucleoside phosphoramidite protecting monomer were purchased from Shanghai GenePharma Co., Ltd.

   The structure of compound containing amino group is shown as following: 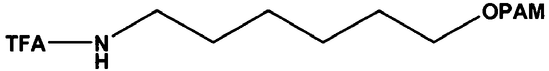
   TFA protecting group on amino group could be removed during protection removal of base group by ammonia.
   The operation steps are: 10~50nmol modified RNA e was dissolved in 40µl 0.5M sodium carbonate/sodium bicarbonate buffer solution (pH= 8.75), 12µl DMSO solution containing 10µmol compound d was added, the mixture was incubated at room temperature for 4h. The obtained nucleic acid crude product was desalted by using NAP-10 gel column and purified with reverse HPLC, it was then desalted by using NAP-10 gel column, and as a result, final product f was obtained. 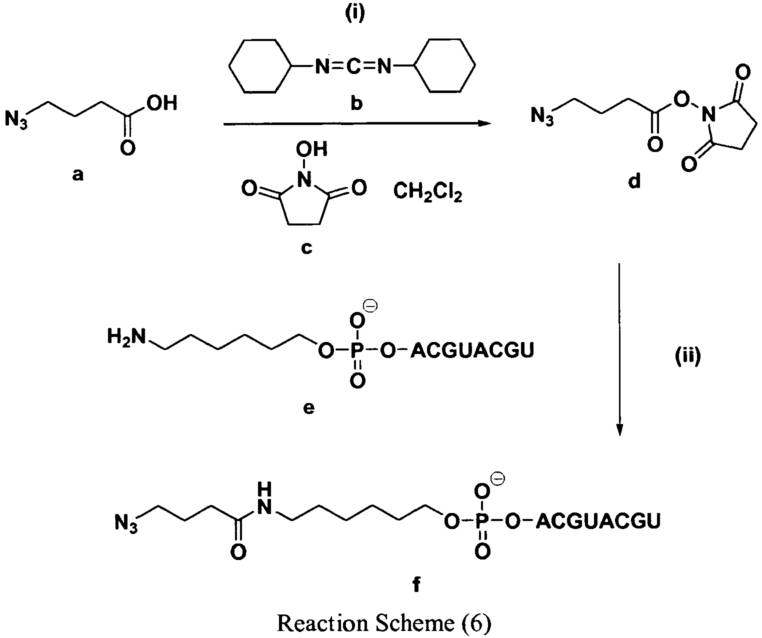
4. Linking reaction was performed according to Reaction Scheme (7):
   TBTA ligand (1.38µmol), sodium vitamin C (2.0µmol) and copper sulfate pentahydrate (0.20µmol) were added sequentially to 950µl 0.2M NaCl buffer solution and mixed. Subsequently, modified single-strand nucleic acid a (2.0nmol), modified single-strand nucleic acid b (2.0nmol) were added, the reaction mixture was incubated at room temperature for 2h. After the reaction was completed, the obtained crude product was first desalted by using NAP-10 gel column and purified with anion exchange HPLC. Product c was desalted by using NAP-10, and analyzed with MALDI-TOF mass chromatography.

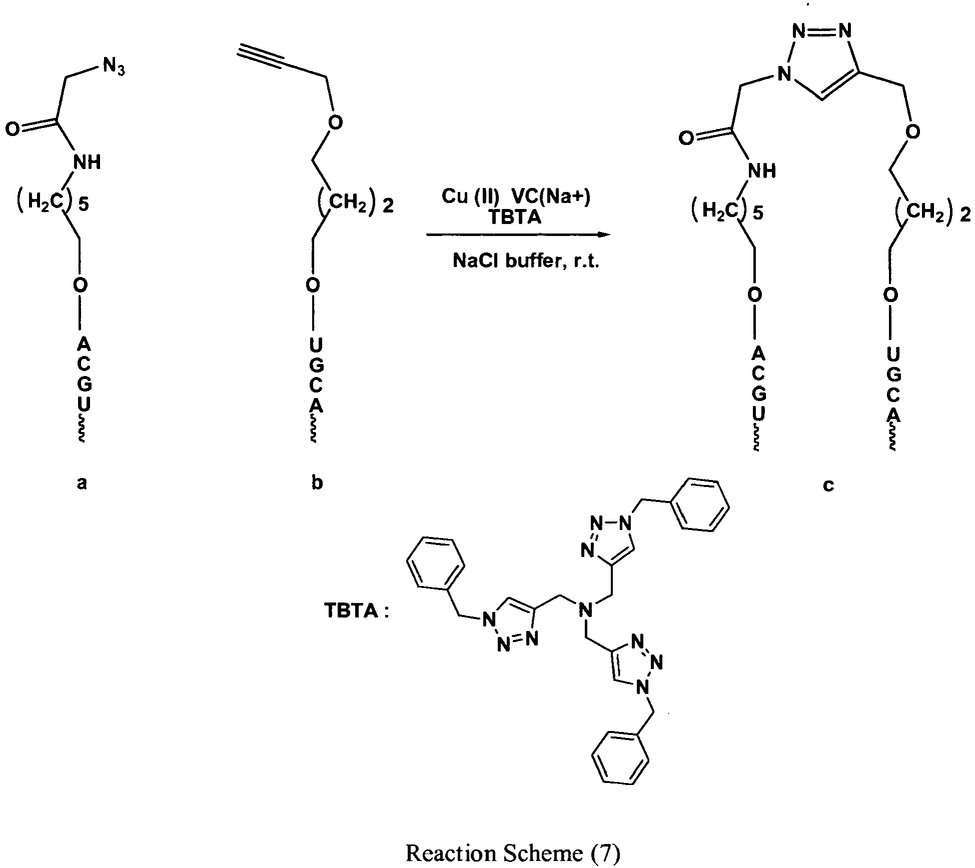

The structure of part of compounds obtained from said reaction and their ¹H NMR data are shown in Table 1, the structure of part of compounds obtained from said reaction and their ³¹P NMR data are shown in Table 2.

**Table 1**

| Structure | ¹H NMR (δ, 400MHz, CDCl₃, ppm) |
|---|---|
| 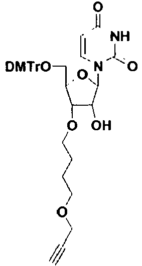 | 1.23~1.27 (t, 4H), 1.62~1.71 (m, 4H), 1.84 (s, 1H), 2.04 (s, 2H), 3.09 (s, 1H), 3.38~3.42 (m, 1H), 3.47∼3.50 (m, 1H), 3.52~3.55 (t, 2H), 3.78 (s, 6H), 3.92~3.94 (m, 2H), 4.08~4.14(m, 4H), 4.24 (s, 1H), 4.29~4.31 (t, 1H), 4.35 (s, 2H), 5.47~5.49 (d, 1H), 5.79~5.80 (d, 1H), 6.81~6.83 (d, 4H), 7.20~7.29 (m, 8H), 7.34~7.36 (t, 2H), 7.75~7.77 (d, 1H). |
| 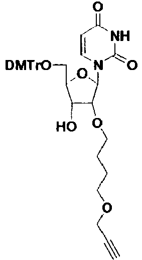 | 1.24~1.27 (t, 4H), 1.62~1.71 (m, 4H), 1.84 (s, 1H), 2.04 (s, 2H), 3.09 (s, 1H), 3.38∼3.42 (m, 1H), 3.47∼3.50 (m, 1H), 3.52∼3.55(t, 2H), 3.78 (s, 6H), 3.92∼3.94 (m, 2H), 4.08~4.14 (m, 4H), 4.24 (s, 1H), 4.29~4.31 (t, 1H), 4.35 (s, 2H), 5.50∼5.52 (d, 1H), 5.77∼5.78 (d, 1H), 6.81∼6.83 (d, 4H), 7.20∼7.29 (m, 8H), 7.33∼7.35 (t, 2H), 7.72∼7.74 (d, 1H). |
| 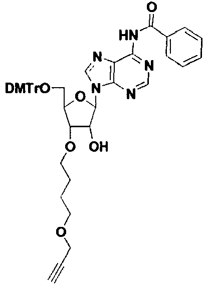 | 0.99~1.02 (t, 4H), 1.62~1.69 (m, 2H), 1.76~1.82 (m, 2H), 2.36 (s, 1H), 2.50∼2.55 (d, 2H), 3.23∼3.25 (d, 1H), 3.41~3.43 (d, 1H), 3.45∼3.48 (t, 2H), 3.77 (s, 6H), 4.04 (s, 2H), 4.36~4.41 (m, 4H), 4.74∼4.77 (t, 1H), 5.93∼5.94 (d, 1H), 6.74∼6.76 (d, 4H), 7.04∼7.08 (t, 2H), 7.12~7.14 (d, 1H), 7.17~7.19 (d, 6H), 7.26 (s, 2H), 7.40∼7.42 (d, 2H), 8.12 (s, 1H), 8.50 (s, 1H). |
| 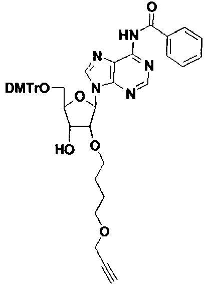 | 1.00∼1.04 (t, 4H), 1.63~1.69 (m, 2H), 1.77~1.82 (m, 2H), 2.36 (s, 1H), 2.50∼2.55 (d, 2H), 3.22∼3.25 (d, 1H), 3.41~3.43 (d, 1H), 3.45∼3.48 (t, 2H), 3.77 (s, 6H), 4.04 (s, 2H), 4.37∼4.42 (m, 4H), 4.76∼4.78 (t, 1H), 5.92∼5.93 (d, 1H), 6.74∼6.76 (d, 4H), 7.06∼7.08 (t, 2H), 7.12~7.14 (d, 1H), 7.17~7.19 (d, 6H), 7.26 (s, 2H), 7.40∼7.42 (d, 2H), 8.11 (s, 1H), 8.51 (s, 1H). |

**Table 2**

| Structure | ³¹P NMR (δ, 122MHz, CDCl₃, ppm) |
|---|---|
| 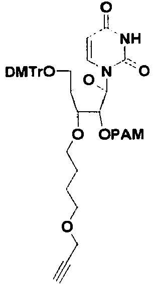 | 146.70, 155.32 |
| 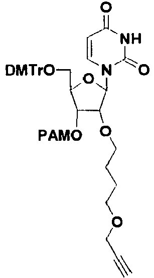 | 146.75, 155.36 |
| 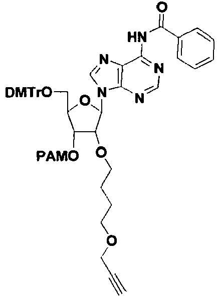 | 147.22, 156.55 |

### Example 2

The present example illustrates the linkage of fluorescent marker molecules

Method for the linkage of fluorescent marker molecules is based on Reaction Scheme (8). Compound b of Reaction Scheme (8) is modified product of fluorescent dye dansyl chloride, and its emission wavelength is 530nm.

TBTA ligand (1.38µmol), sodium vitamin C (2.0µmol) and copper sulfate pentahydrate (0.20µmol) were added sequentially to 950µl 0.2M NaCl buffer solution and mixed. Subsequently, modified double-stranded nucleic acid a (2.0nmol) and fluorescent dye molecule b (2.0nmol) were added, the reaction mixture was incubated at room temperature for 2h. After the reaction was completed, the obtained crude product was first desalted by using NAP-10 gel column and purified with anion exchange HPLC. Product c was desalted by using NAP-10, and analyzed with MALDI-TOF mass chromatography or fluorescence detector.

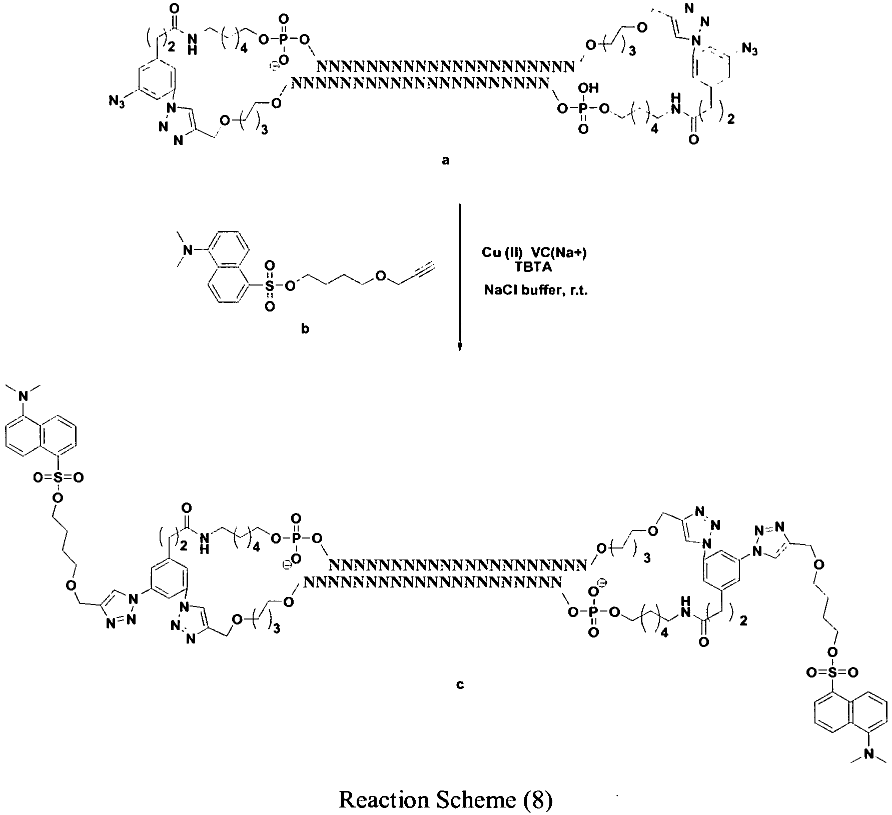

The structure of TBTA of Reaction Scheme (8) is shown as following:

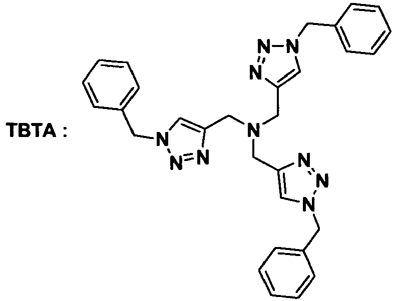

In Reaction Scheme (8), NNNN......NNNN indicates two strands of siRNA, the sequence of sense strand is: 5' GUA UGA CAA CAG CCU CAA GTT 3'; and the sequence of antisense strand is: 5' CUU GAG GCU GUUGUC AUA CTT 3'.

### Example 3

The present example illustrates the determination the interfering effect of ziRNA on the expression of target genes obtained from Example 1 and Example 2.

### (1) Incubation of HEK293 cells (human embryo kidney line)

Using DMEM complete medium containing 10% fetal bovine serum and 2mM L-glutamine, HEK293 cells (obtained from Institute of Molecular Medicine, Peking University) were inoculated on a 6-well cell culture plate with a density of 5 x 10⁶ cell/well, and then were incubated in a incubator containing 5% CO₂ at 37°C, medium was renewed every 48h.

### (2) Transfection of ziRNA

ziRNA obtained from Example 1 and ziRNA obtained from Example 2 were transfected with Lipofectamine^{™} 2000 liposome (Invitrogen Company), respectively, liposome without the addition of ziRNA was used as negative control, and liposome with the addition of siRNA was used as positive control. The detailed operation steps were as follows:
ziRNA was dissolved in RNA enzyme-free abacterial water, and as a result 20µmol/L ziRNA solution was prepared. HEK293 cells were inoculated to a 24-well plate and diluted with OptiMEM I low serum medium (Invitrogen Company, 31985-062) until the concentration reached 8 x 10⁵ cell/ml, 500µl per well. 3µl ziRNA solution (20µmol/L) was diluted with 50µl Opti-MEM I low serum medium (Invitrogen Company, 31985-062), 1µl Lipofectamine^{™} 2000 liposome was diluted with 50µl Opti-MEM I low serum medium (Invitrogen Company, 31985-062), subsequently, the two obtained solutions were mixed, after incubated at room temperature for 5min, the mixed solution was allowed to stand at room temperature for 20min, 100µl said solution mixture was added to said 24-well plate containing inoculated cell. The final concentration of ziRNA was 100nM. Cells were incubated at 37°C for 4h, subsequently, 1ml DMEM complete medium containing 10% fetal bovine serum, 2mM L-glutamine, 100U/ml penicillin and 100µg/ml streptomycin was added, and then it was incubated at 37°C for 24h.

### (3) Determination of the inhibition of expression of target genes with Realtime method

The expression of GAPDH gene mRNA of HEK293 cell transfected with ziRNA of Example 1 and ziRNA of Example 2 was determined with Realtime-PCR, HEK293 cells untransfected with ziRNA were used as negative control and transfected with siRNA (sequence of sense: 5' GUA UGA CAA CAG CCU CAA GTT 3'; sequence of antisense: 5' CUU GAG GCU GUUGUC AUA CTT 3') was used as positive control.

The detailed steps were: HEK293 cells transfected with ziRNA and the controls were lysed with 1ml Trizol (BIGCOL Company), the total RNA was extracted by the following steps: transfected cells were incubated in an incubator containing 5% CO₂ at 37°C for 24h, subsequently, it was centrifuged, cells were collected, and washed with 2ml precooled PBS containing 137mol/L NaCl, 2.7mmol/L KCI, 4.3mmol/L Na₂HPO₄ and 1.4mmol/L KH₂PO₄; 1ml Trizol was added to each well, it was then allowed to stand at room temperature for 5min, cells were lysed; the lysed materials were transferred to a 1.5ml EP tube; 200µl chloroform was added, shaken in hand for 15 seconds and allowed to stand at room temperature for 3min; it was then centrifuged at 4°C for 15min at 14000rpm; 500µl supernatant liquid was added to a new EP tube, 500µl isopropanol was added, it was allowed to stand at room temperature for 10min; it was then centrifuged at 4°C for 10min at 12000rpm, supernatant liquid was removed, the precipitates were washed with 1ml 75% ethanol, it was centrifuged at 4°C for 5min at 7600rpm, the supernatant liquid was removed, RNA precipitates were dried at room temperature for 10min; subsequently 20µl ddH₂O was added to dissolve the precipitates.

2 units DNaes I (RNase-free) (TakaRa Company) were added to said DEPC water containing RNA, it was then allowed to stand at 37°C for 30min to remove residue DNA of total RNA. After treated with DNase I, total RNA was purified with PureLink Micro-to-Midi Total RNA Purification Kit (Invitrogen Company) by the following steps: 20µl 70% ethanol was added to total RNA, the mixture was shaken well, subsequently the mixture was transferred to purification column, centrifuged at room temperature for 15 seconds at 12000rpm, the filtrate was removed, 500µl wash buffer II (TakaRa Company) was added, it was then centrifuged at room temperature for 15 seconds at 12000rpm, the filtrate was removed, and then 500µl wash buffer II (TakaRa Company) was added, it was centrifuged at room temperature for 15 seconds at 12000rpm, the filtrate was removed, centrifuged at room temperature for 1min at 12000rpm, the purification column was transferred to RNA collecting tube, 30µl DEPC water was added, it was allowed to stand at room temperature for 1min, centrifuged at room temperature for 2min at 13000rpm, and the RNA sample was stored at -80°C.

The purified total RNA underwent reverse transcription reaction, by using M-MLV reverse transcriptase from Promega Company and by the following steps: 1µg purified total RNA was mixed with 0.5µg Oligo dT in a tube, DEPC water was added until the total volume of 16.25µl was reached, the tube was then heated at 70°C for 5min; subsequently the tube was rapidly cooled to 0°C, buffer solution (5×MLV buffer 5µl, 10mM Dntp 1.25µl, RNasin 0.5µl, M-MLV 1µl) was added, it was then incubated at 42 °C for 1h, as a result, cDNA was obtained.

The obtained cDNA was used as template for PCR reaction, and Real-time PCR was performed. Real-time PCR reaction system was: 17.5µl ddH₂O, 0.5µl 10mM Dntp, 2.5µl 10×Taq buffer, 0.5µl Taq, 0.5µl F primer, 0.5µl R primer, 1µl Syber Green I, and 2µl cDNA; PCR reaction conditions were: 2min at 94°C, 15sec at 94°C, 30sec at 60°C, and 40 cycles in total. Meanwhile β-actin was used as inner reference, and the inhibitory rate of ziRNA was calculated according to the following equation.

Inhibitory rate of ziRNA = [1-(copy number of GAPDH genes after transfected with ziRNA /copy number of β-actin after transfected with ziRNA)/(copy number of GAPDH genes in control well /copy number of β-actin in control well)] x 100%.

Based on said equation, the inhibitory rate of siRNA to GAPDH was 91 %; the inhibitory rate of transfected ziRNA of Example 1 and ziRNA of Example 2 to GAPDH was 92% and 89%, respectively.

It can be seen from said results that ziRNA of present invention could effectively inhibit the expression of GAPDH genes.

### Example 4

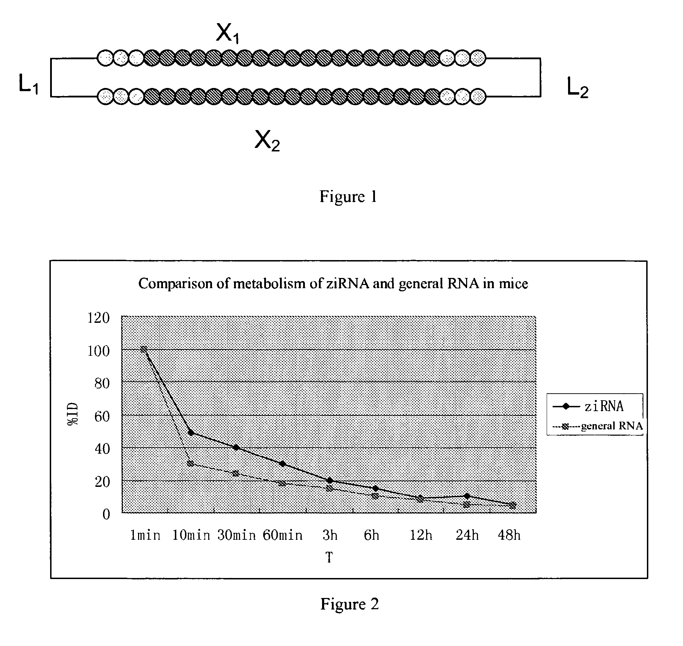

The present example illustrates the determination of pharmacokinetics of ziRNA of Example 1. ziRNA of Example 1 and general siRNA (sequence of sense strand: 5' GUA UGA CAA CAG CCU CAA GTT 3'; sequence of antisense strand: 5' CUU GAG GCU GUUGUC AUA CTT 3') were labeled as 2µCi/µg with ³²P end labeling method, 60 st Kunming mice (weight 20-25mg, male or female) were used during the experiments, the radioactively labeled ziRNA or general siRNA was injected to mice with a dosage of 10mg/kg weight. Before injection, and 1 min, 10min, 30 min, 60min, 3h, 6h, 12 h, 24 h, 48h after injection, 3 mice were chosen, respectively, blood was taken from orbit vein, and the blood plasma was separated. Radioactivity of blood plasma was determined, and the results were shown in Figure 2. It could be seen from Figure 2 that the residence time of ziRNA in blood serum is longer than that of general siRNA.

### Example 5

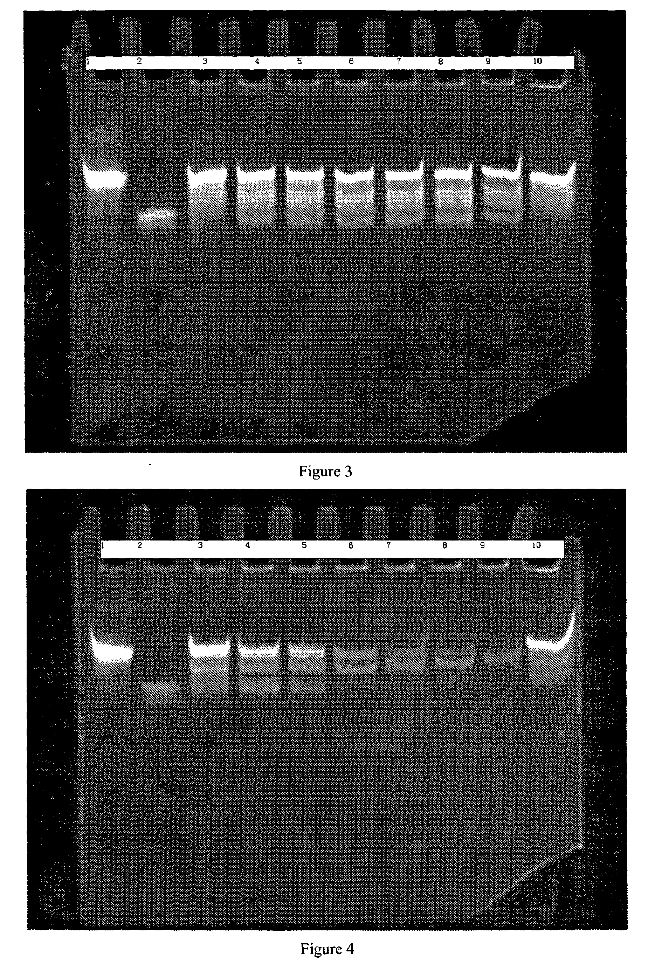

The present example illustrates the determination of stability of ziRNA of Example 1. ziRNA of Example 1 and 10% blood serum were incubated for 1min, 30min, 1.5h, 3h, 6h, 12h and 24h, respectively, and then underwent 20% PAGE electrophoresis to determine the stability of ziRNA in blood serum, and the results were shown in Figure 3. The numerals in Figure 3 represent: 1: ziRNA; 2: single strand RNA; 3: 1min; 4: 30min; 5: 1.5h; 6: 3h; 7: 6h; 8: 12h; 9: 24h; 10: RNA.

General siRNA (sequence of sense strand: 5' GUA UGA CAA CAG CCU CAA GTT 3'; sequence of antisense strand: 5' CUU GAG GCU GUUGUC AUA CTT 3') and 10% blood serum were incubated for 1min, 30min, 1.5h, 3h, 6h, 12h and 24h, respectively, and then underwent 20% electrophoresis to determine the stability of general siRNA in blood serum, and the results were shown in Figure 4. The numerals in Figure 4 represent: 1: general siRNA; 2: single strand RNA; 3: 1min; 4: 30min; 5: 1.5h; 6: 3h; 7: 6h; 8: 12h; 9: 24h; 10: RNA.

By comparing Figure 3 and Figure 4, it can be seen that double-stranded ziRNA is more stable in blood serum than siRNA. ziRNA was present stabily in blood serum for more than 24h, however, general siRNA degraded considerably after incubated in blood serum for 30min, and no double-stranded RNA was observed after 6h.

### Example 6

The present example illustrates the preparation of complex molecule interfering the expression of target genes.

Complex molecule was prepared in the same manner of Example 1, except that preparation of nucleic acid containing alkynkl group in step 2 was as follows:
(1) Synthesis of 2'-Deoxy-1'α/β-propynyloxy-D-ribofuranose 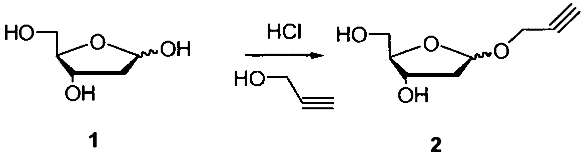
   5.2g (4mmol) compound 1 was added to a 250ml flask in an ice bath, 3.9g (7.3mmol) propargyl alcohol was added, and 1.27g concentrated HCl was dropped under stirring, the materials were then mixed at room temperature (20°C) for 3h. TLC was performed to monitor the end of reaction, 20ml redistilled dried pyridine was added, the solvent was evaporated, column chromatography was performed (petroleum ether: ethyl acetate: methanol = 1: 1: 0.25) and 6.6g white solid compound 2 was obtained. The yield was 75%. ¹H NMR(400M, CDCl₃) δ ppm 5.22(1H, m, H1'), 4.22-4.07(2H, m, OCH₂), 3.91-3.86(2H, m, H4', H5'a), 3.65-3.43 (2H, m, H5'b, H3'), 2.45(1H, t, CH), 1.96-1.98(2H, m, H2'). ESI-MS[M+ Na]⁺, 195.0628.
(2) Synthesis of 2'-Deoxy-1'α/β-propynyloxy-3'-di-O-acetyl-D-ribofuranose 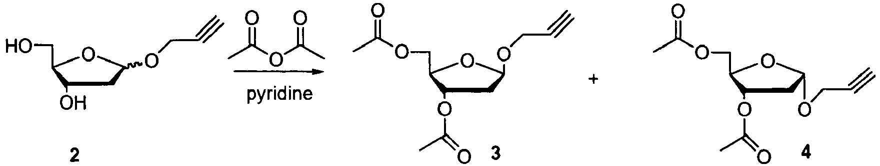
   1.72g (1mmol) compound 2 was dissolved in 10ml dried pyridine, and it was added to a 100ml flask together with 3.06g (3mmol) acetic anhydride, the temperature was rapidly increased to 150°C and refluxed for 5min, TLC was performed to monitor the end of reaction. The reaction solution was added to 10ml water, extracted with dichloromethane (15ml x 3) and dried. The solvent was evaporated, column chromatography (petroleum ether: ethyl acetate= 5:1) was performed several times, 0.86g light yellow oil compound 3 and 1.60g white crystal compound 4 were obtained. The total yield was 91%. ¹H NMR, α-anomer: ¹H NMR(400M, CDCl₃) δ ppm 5.09(1H, t, H1'), 4.24-4.21(2H, m, H4', H3'), 3.88-3.76(4H, m, H5', OCH₂), 2.45(1H, s, CH), 1.97-1.91(8H, m, COCH₃×2, H2'a, b);β-anomer: ¹H NMR(400M, CDCl₃) δ ppm 5.23(1H, m, H1'), 4.32-4.16(3H, m, H4', H3', H5'a), 3.76-3.62(3H, m, H5'b, OCH₂), 2.46(1H, m, CH), 2.23-1.95(8H, m, COCH₃×2, H2'a, H2'b). ESI-MS[M+ Na]⁺, 279.0839.
(3) Synthesis of 2'-Deoxy-1'β-propynyloxy-D-ribofuranose 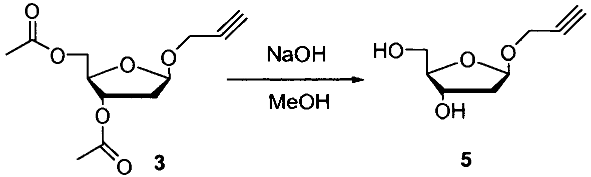
   500mg (1.95mmol) compound 3 was added to a 50ml flask, it was mixed and dissolved in 5ml methanol, subsequently 4ml NaOH (10%) methanol solution was added, the reaction mixture was mixed at room temperature for 5min, TLC was performed to monitor the end of reaction. 10ml water was added, part of methanol was evaporated, the reaction solution was extracted with ethyl acetate (15ml x 3) for three times and dried, the solvent was evaporated, the product was recrystallized with methanol, as a result, 320g white solid compound 5 was obtained, the yield was 92%. ¹H NMR(400M, CDCl₃) δ ppm 5.23(1H, m, H1'), 4.30-4.23(2H, m, OCH₂), 4.19(1H, m, H4'), 3.86(1H, m, H5'a), 3.65-3.61(2H, m, H5'b, H3'), 2.47(1H, dt, CH), 2.17-1.95(2H, m, H2'). ESI-MS[M+ Na]⁺, 195.0628.
(4) Synthesis of 2'-Deoxy-1'β-propynyloxy-5'-*O*-dimethoxytrityl-D-ribofuranose 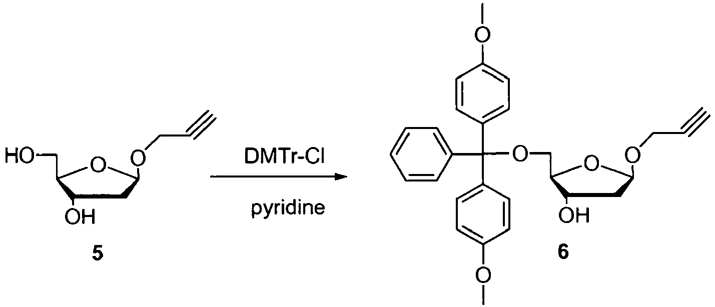
   320mg (1.9mmol) compound 5 was added to a 50ml flask, it was evaporated with 5ml dried pyridine for three times to remove water. 5ml dried pyridine was added to dissolve the compound 5, 1.352g DMTr-Cl (4mmol) was added, the mixture was mixed at room temperature for 0.5h, TLC was performed to monitor the end of reaction. 3ml absolute methanol was added, and the mixture was mixed for another 5min. The reaction solution was added to 30ml 5% sodium bicarbonate aqueous solution. The mixed solution was extracted with dichloromethane (15ml x 3) and dried. The solvent was evaporated, and column chromatography (petroleum ether: ethyl acetate= 3:1) was performed, 600mg white flocculate solid compound 6 was obtained, the yield was 71%. H NMR(400M, CDCl₃) δ ppm 7.56-7.21(9H, m, Ar-H), 6.84(4H, m, Ar-H), 5.23(1H, m, H1'), 4.34-4.11(3H, OCH₂ ,H4'), 3.81(6H, s, OCH₃×2), 3.63-3.51(3H, m, H3'H5'), 2.45(1H, m, CH), 2.36-2.06(2H, m, H2'). ESI-MS[M+ Na]⁺, 497.1935.
(5) Synthesis of 2'-Deoxy-1'β-propynyloxy-3'-*O*-(2-Cyanoethyl N,N-Diisopropylphosphoramidite)-5'-O-dimethoxytrityl-D-ribofuranose 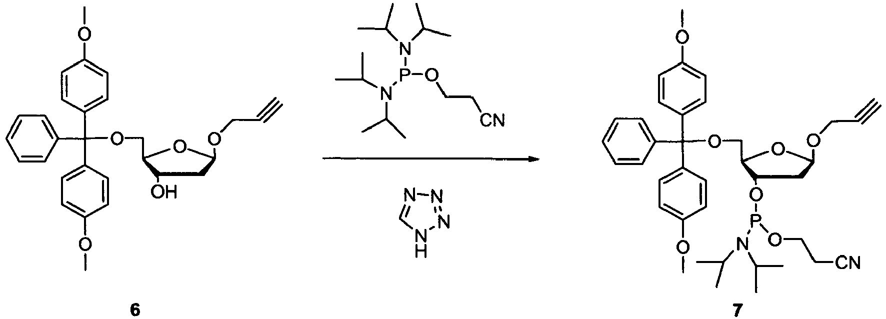
   160mg (2.28mmol) 1-H tetrazole and 760mg (5.56mmol) phosphoramide were dissolved in 5ml redistilled dichloromethane in a 50ml two-neck flask under N₂ atmosphere. The mixture was stirred for 5min, subsequently 4ml redistilled dichloromethane containing 600mg (1.27mmol) compound 6 was dropped, it was stirred at room temperature for 10h under N₂ atmosphere, TCL was performed to monitor the end of reaction, solvent was evaporated (petroleum ether: ethyl acetate = 5: 1) and then flash column chromatography was carried out, as a result, 620mg white flocculate solid compound 7 was obtained, the yield was 82%. ¹H NMR(400M, CDCl₃) δ ppm 7.56-7.21(9H, m, Ar-H), 6.84(4H, m, Ar-H), 5.36(1H, m, H1'), 4.37-4.23(4H, m, OCH₂C, H4', POCH₂a), 3.80(6H, s, OCH₃×2), 3.63-3.51(3H, m, H3', H5'), 3.36-3.07(2H, m, CH×2), 2.65(1H, m, POCH₂b), 2.56(1H, m, CCH), 2.37-2.06(2H, m, H2'), 1.23(12H, m, 4×CH₃); ³¹P-NMR(162M, CDCl₃): δ ppm 148.3,147.4; ¹³C NMR(CDCl₃, 100MHz) δ ppm 158.52, 145.89, 136.92, 130.52, 128.56, 127.65, 126.73, 117.72, 112.99, 96.752, 86.37, 79.69, 77.41, 77.10, 76.78, 73.90, 71.16, 66.73, 63.89, 57.20, 55.22, 53.90, 43.35, 32.09, 24.74, 20.38. ESI-MS[M+ Na]⁺, 697.3013.

## Claims

1. A complex molecule interfering the expression of target genes, wherein the complex molecule contains two siRNA strands X₁ and X₂ having at least 80% complementarity, the 5' end of X₁ and 3' end of X₂ are linked through a non-nucleic acid molecule L₁, and the 5' end of X₂ and 3' end of X₁ are linked through a non-nucleic acid molecule L₂, wherein the non-nucleic acid molecule L₁ and non-nucleic acid molecule L₂ are independently represented by Formula I: 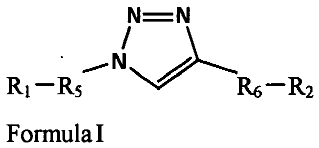 the groups R₁, and R₂ are independently carboxyl group, amino group or mercapto group; R₅ is 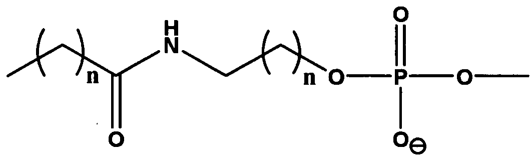 or 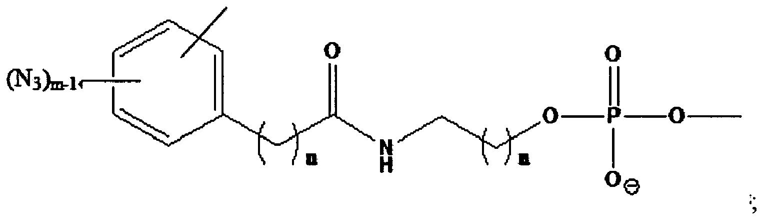 R₆ is one of the following groups: 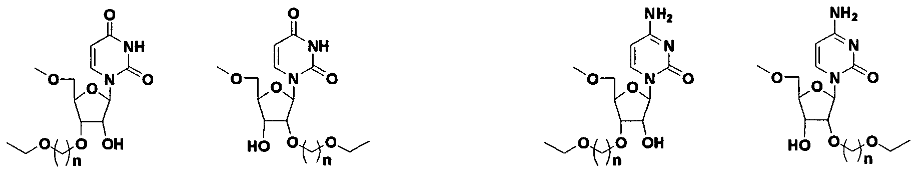 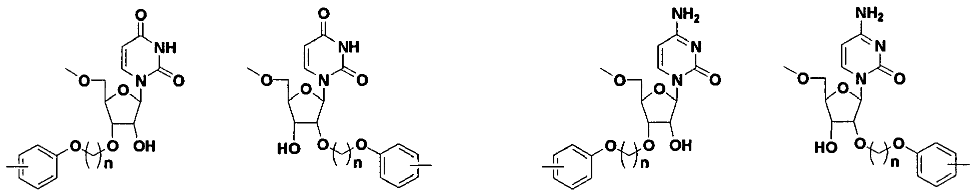 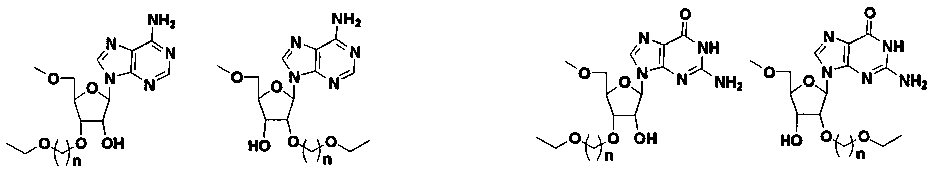 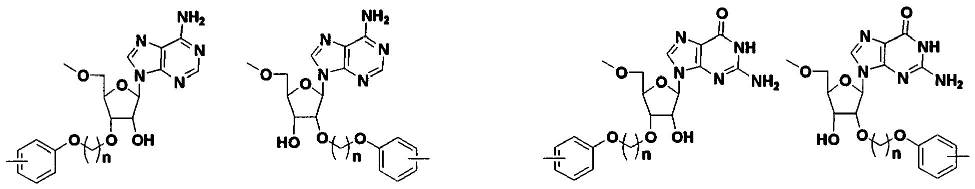 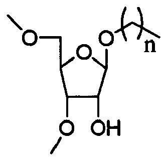 and in the groups, n is independently an integer between 0~10, and m is independently an integer between 1~5.

2. The complex molecule of Claim 1, wherein the non-nucleic acid molecule L₁ is covalently linked to phosphate group or hydroxyl group of 5' end of X₁ and phosphate group or hydroxyl group of 3' end of X₂, and the non-nucleic acid molecule L₂ is covalently linked to phosphate group or hydroxyl group of 5' end of X₂ and phosphate group or hydroxyl group of 3' end of X₁.

3. The complex molecule of Claim 1, wherein the non-nucleic acid molecule L₁ and/or L₂ is linked with one or more selected from cell target recognizing molecule, lipid molecule capable of improving the cell penetration, and fluorescent marker molecule.

4. The complex molecule of Claim 3, wherein
R₅ is 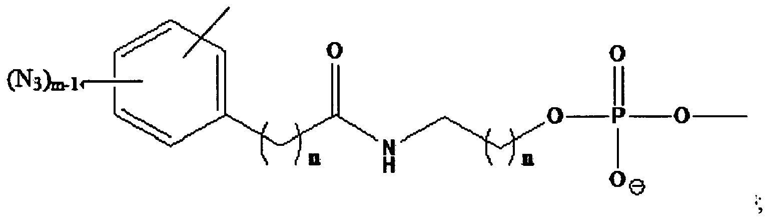 in the groups, n is independently an integer between 0~10, and m is independently an integer between 2∼5,
one or more selected from the cell target recognizing molecule, lipid molecule capable of improving the cell penetration, and fluorescent marker molecule is linked to azide group N₃ of R₅.

5. The complex molecule of Claim 3 or Claim 4, wherein the chemical structure of the cell target recognizing molecule, lipid molecule capable of improving the cell penetration or fluorescent marker molecules is 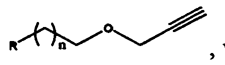 wherein R is one or more of cell target recognizing group, lipid group capable of improving the cell penetration or fluorescent marker group, and the alkynyl group in the chemical structure is linked to L₁ and/or L₂.

6. The complex molecule of Claim 5, wherein the alkynyl group is linked to azide group N₃ of R₅ of L₁ and/or L₂, so as to form

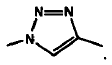

7. A method for the preparation of the complex molecule interfering the expression of target genes of Claim 1, wherein it includes: preparing two modified siRNA strands X₁ and X₂, having at least 80% complementarity, both 5' and 3' ends of the two modified siRNA strands having a linkable group; linking the linkable groups of 5' and 3' ends of X₁ to the linkable groups of 3' and 5' ends of X₂, respectively, so that the 5' end of X₁ and 3' end of X₂ are linked through a non-nucleic acid molecule L₁, and the 5' end of X₂ and 3' end of X₁ are linked through a non-nucleic acid molecule L₂.

8. The method of Claim 7, wherein the method includes: fixing a3 and a3' respectively, synthesizing X₁ on a3 from 3' end to 5' end, and synthesizing X₂ on a3' from 3' end to 5' end, such that a3-X₁ and a3'-X₂ are obtained; introducing a1 and a1' to 5' end of a3-X₁ and a3'-X₂, respectively, to form a1-X₁-a3 and a1'-X₂-a3'; introducing a2 and a2' to a1-X₁-a3 and a1' -X₂-a3', respectively, to form a2-a1-X₁-a3 and a2'-a1'-X₂-a3'; and then, annealing a2-a1-X₁-a3 and a2'-a1'-X₂-a3', subjecting a2 of a2-a1-X₁-a3 and a3' of a2'-a1'-X₂-a3' to a linking reaction and subjecting a3 of a2-a1-X₁-a3 and a2' of a2'-a1'-X₂-a3' to a linking reaction, such that the complex molecule is formed, wherein
a1 and a1' independently are 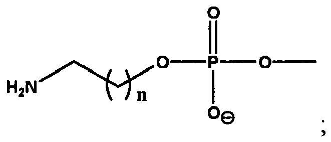 a2 and a2' independently are 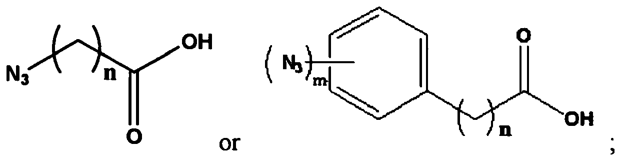 a2-a1 and a2'-a1' independently are 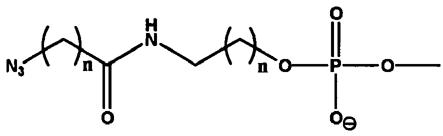 or 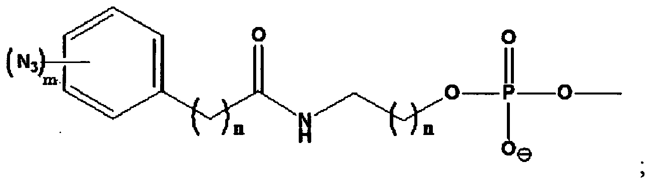 a3 and a3' are independently one of the following groups: 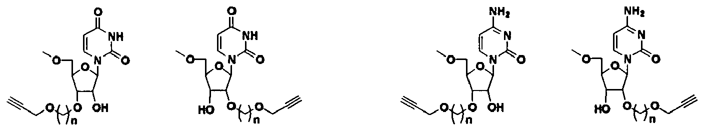 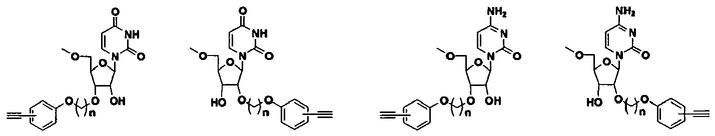 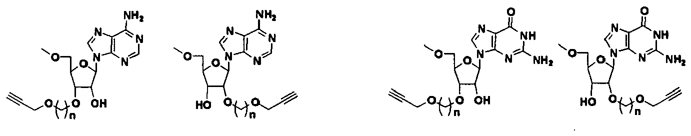 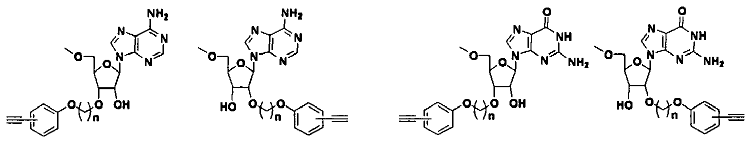 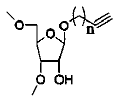 and in the groups, n is independently an integer between 0∼10, and m is independently an integer between 1~5.

9. The method of Claim 8, wherein a2 and/or a2'are 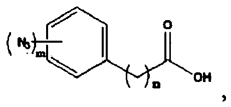 in which m is an integer between 2∼5, and n is an integer between 0~10, and the method further includes linking a2 and/or a2' with one or more of cell target recognizing molecule, lipid molecule capable of improving the cell penetration and fluorescent marker molecule after a2 of a2-a1-X1-a3 and a3' of a2'-a1'-X2-a3' are linked and a3 of a2-a1-X1-a3 and a2' of a2'-a1'-X2-a3' are linked, wherein the cell target recognizing molecule, lipid molecule capable of improving the cell penetration or fluorescent marker molecule is linked to azide group N₃ of a2 and/or a2'.

10. The method of Claim 9, wherein the chemical structure of said cell target recognizing molecule, lipid molecule capable of improving the cell penetration or fluorescent molecule is 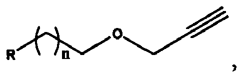 wherein R is one or more of cell target recognizing group, lipid group capable of improving the cell penetration and fluorescent marker group, and the alkynyl group is linked to azide group N₃ of a2 and/or a2', so as to form

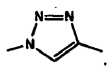

11. The method of Claim 7, wherein the method includes: fixing a4 and a4' respectively, synthesizing X₁ on a4 from 3' end to 5' end, and synthesizing X₂ on a4' from 3' end to 5' end, such that a4-X₁ and a4'-X₂ are obtained; introducing a5 and a5' to 5' end of a4-X₁ and a4'-X₂, respectively, to form a5-X₁-a4 and a5'-X₂-a4'; first linking a6 to one strand of a5-X₁-a4 and a5'-X₂-a4', annealing the two strands, and then linking a6 to the other strand, such that the complex molecule is formed, wherein a4, a4', a5 and a5' are one of the following groups: 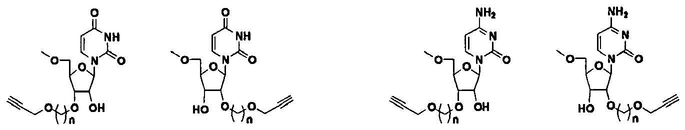 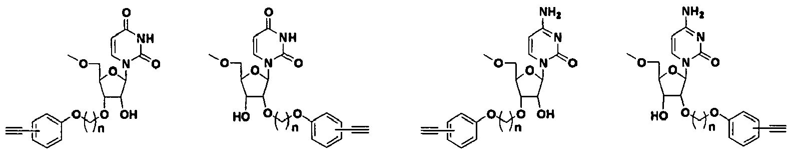 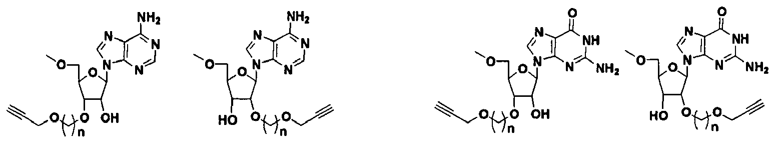 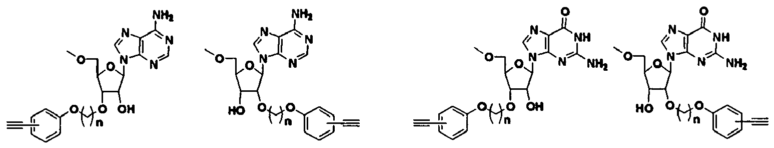 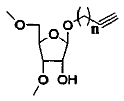 wherein n is an integer between 0~10;
a6 is N₃-(CH₂)ₚ-N₃, wherein p is an integer between 2~12.

12. Use of the complex molecule of Claim 1 for improving the chemical stability of siRNA.

13. Use of the complex molecule of Claim 1 for improving the remaining time in the blood of siRNA.

## Patentansprüche

1. Komplexes Molekül, das in die Expression von Zielgenen eingreift, wobei das komplexe Molekül zwei siRNA-Stränge X₁ und X₂ enthält, die zu mindestens 80 % komplementär sind, wobei das 5'-Ende von X₁ und das 3'-Ende von X₂ über ein Nicht-Nukleinsäuremolekül L₁ verbunden sind und wobei das 5'-Ende von X₂ und das 3'-Ende von X₁ über ein Nicht-Nukleinsäure-Molekül L₂ verbunden sind, wobei das Nicht-Nukleinsäure-Molekül L₁ und das Nicht-Nukleinsäure-Molekül L₂ unabhängig voneinander der Formel I entsprechen: 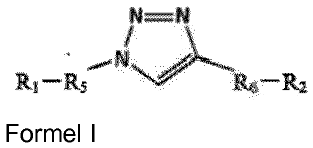 wobei die Gruppen R₁ und R₂ unabhängig voneinander für eine Carboxylgruppe, Aminogruppe oder Mercaptogruppe stehen;
wobei R₅ 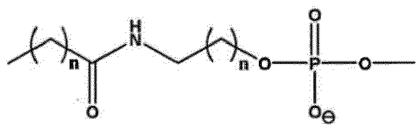 oder 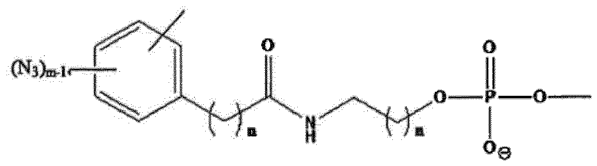 entspricht,
wobei R₆ einer der folgenden Gruppen entspricht: 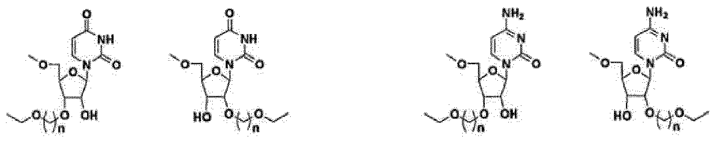 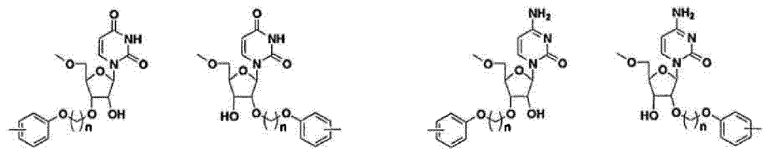 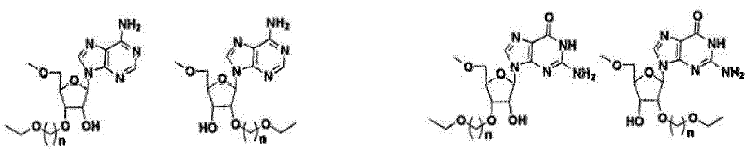 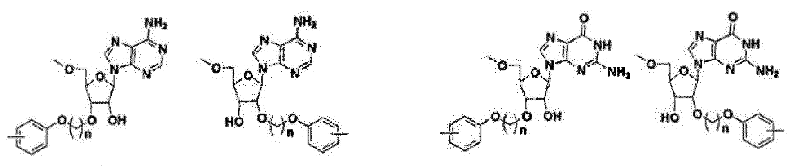 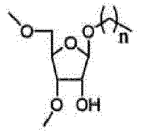 und wobei n in den Gruppen auf unabhängige Weise für eine ganze Zahl zwischen 0~10 steht, und m auf unabhängige Weise für eine ganze Zahl zwischen 1~5 steht.

2. Komplexes Molekül nach Anspruch 1, wobei das Nicht-Nukleinsäuremolekül L₁ kovalent an die Phosphatgruppe oder Hydroxylgruppe des 5'-Endes von X₁ und die Phosphatgruppe oder Hydroxylgruppe des 3'-Endes von X₂ gebunden ist und wobei das Nicht-Nukleinsäuremolekül L₂ kovalent an die Phosphatgruppe oder Hydroxylgruppe des 5'-Endes von X₂ und die Phosphatgruppe oder Hydroxylgruppe des 3'-Endes von X₁ gebunden ist.

3. Komplexes Molekül nach Anspruch 1, wobei das Nicht-Nukleinsäuremolekül L₁ und/oder L₂ mit einem oder mehreren Elementen verbunden ist/sind, die aus einem Molekül für die Erkennung von Zielzellen, aus einem Lipidmolekül, das zur Verbesserung der Zellpenetration befähigt ist, und aus einem fluoreszierenden Markermolekül ausgewählt ist/sind.

4. Komplexes Molekül nach Anspruch 3, wobei
wobei R₅ 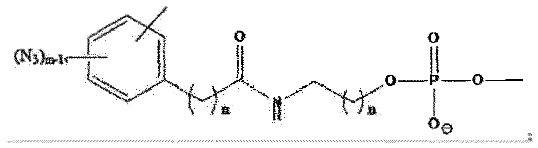 entspricht, wobei n in den Gruppen auf unabhängige Weise für eine ganze Zahl zwischen 0~10 steht, und m auf unabhängige Weise für ganze Zahl zwischen 2-5 steht.
wobei eines oder mehrere der Elemente, die aus dem Molekül für die Erkennung von Zielzellen, aus dem Lipidmolekül, das zur Verbesserung der Zellpenetration befähigt ist, und aus dem fluoreszierenden Markermolekül ausgewählt ist/sind, mit der Azidgruppe N₃ von R₅ verbunden ist/sind.

5. Komplexes Molekül nach Anspruch 3 oder Anspruch 4, wobei die chemische Struktur des Moleküls für die Erkennung von Zielzellen, des Lipidmoleküls, welches zur Verbesserung der Zellpenetration befähigt ist, oder des fluoreszierenden Markermoleküls gleich 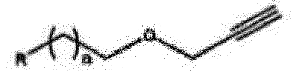 ist, wobei R für eines oder mehrere Elemente von einer Gruppe für die Erkennung von Zielzellen, einer Lipidgruppe, welche zur Verbesserung der Zellpenetration befähigt ist, oder einer fluoreszierenden Markergruppe steht, und wobei die Alkinylgruppe in der chemischen Struktur an L₁ und/oder L₂ gebunden ist.

6. Komplexes Molekül nach Anspruch 5, wobei die Alkinylgruppe derart an die Azidgruppe N₃ des R₅ von L₁ und/oder L₂ gebunden ist, sodass 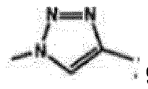 gebildet wird.

7. Verfahren zur Herstellung des komplexen Moleküls nach Anspruch 1, das in die Expression von Zielgenen eingreift, wobei das Verfahren Folgendes umfasst: Herstellen zweier modifizierter siRNA-Stränge X₁ und X₂, die zu mindestens 80 % komplementär sind, wobei sowohl die 5'-Enden als auch die 3'-Enden der beiden modifizierten siRNA-Stränge eine bindungsfähige Gruppe aufweisen; Verbinden der bindungsfähigen Gruppen der 5'- und 3'-Enden von X₁ mit den 3'-beziehungsweise 5'-Enden von X₂, sodass das 5'-Ende von X₁ und das 3'-Ende von X₂ über ein Nicht-Nukleinsäure-Molekül L₁ verbunden sind und das 5'-Ende von X₂ und das 3'-Ende von X₁ über ein Nicht-Nukleinsäure-Molekül L₂ verbunden sind.

8. Verfahren nach Anspruch 7, wobei das Verfahren Folgendes umfasst:
Immobilisieren von a3 beziehungsweise a3', Synthetisieren von X₁ ausgehend von a3 vom 3'- zum 5'-Ende und Synthetisieren von X₂ ausgehend von a3' vom 3'- zum 5'-Ende, sodass a3-X₁ und a3'-X₂ erhalten werden; Anfügen von a1 und a1' an das 5'-Ende von a3-X₁ beziehungsweise a3'-X₂, um a1-X₁-a3 und a1'-X₂-a3' zu bilden;
Anfügen von a2 und a2' an a1-X₁-a3 beziehungsweise a1'-X₂-a3', um a2-a1-X₁-a3 und a2'-a1'-X₂-a3' zu bilden; und anschließend Hybridisieren von a2-a1-X₁-a3 und a2'-a1'-X₂-a3', Einleiten einer Bindungsreaktion, die a2 von a2-a1-X₁-a3 und a3' von a2'-a1'-X₂-a3' betrifft, und Einleiten einer Bindungsreaktion, die a3 von a2-a1-X₁-a3 und a2' von a2'-a1'-X₂-a3' betrifft, sodass sich das komplexe Molekül bildet, wobei
a1 und a1' unabhängig voneinander 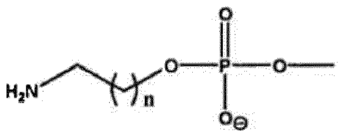 entsprechen,
a2 und a2' unabhängig voneinander 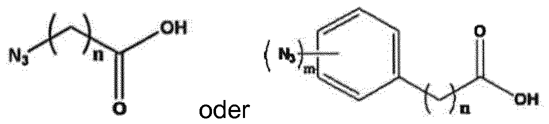 entsprechen,
a2-a1 und a2'-a1' unabhängig voneinander 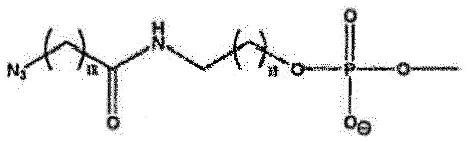 oder 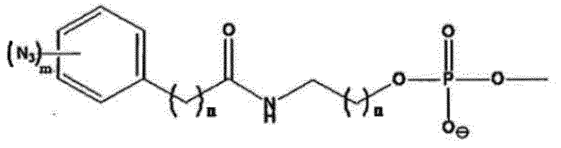 entsprechen,
a3 und a3' unabhängig voneinander einer der folgenden Gruppen entsprechen: 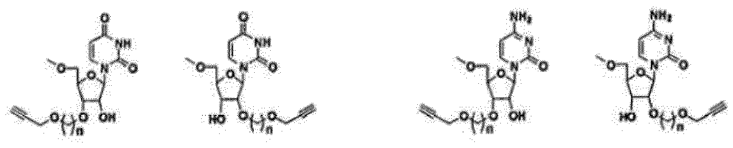 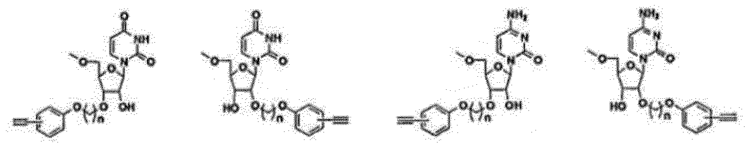 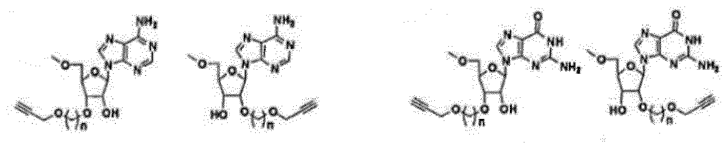 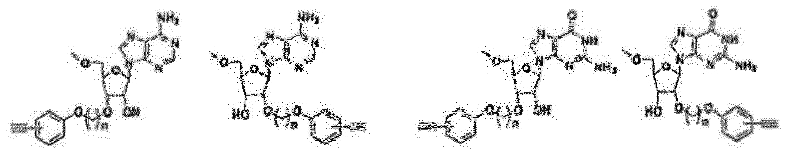 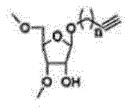 und wobei n in diesen Gruppen auf unabhängige Weise für eine ganze Zahl zwischen 0~10 steht, und m auf unabhängige Weise für eine ganze Zahl zwischen 1~5 steht.

9. Verfahren nach Anspruch 8, wobei a2 und/oder a2' 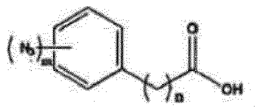 entsprechen, wobei m ist eine ganze Zahl zwischen 2-5 und n eine ganze Zahl zwischen 0~10 ist, und wobei das Verfahren weiterhin, nach dem Verbinden des a2 von a2-a1-X1-a3 und des a3' von a2'-a1'-X2-a3' sowie dem Verbinden des a3 von a2-a1-X1-a3 und des a2' von a2'-a1'-X2-a3' das Verbinden von a2 und/oder a2' mit einem oder mehreren Elementen von einem Molekül für die Erkennung von Zielzellen, einem Lipidmolekül, das zur Verbesserung der Zellpenetration befähigt sind, und einem fluoreszierenden Markermolekül umfasst, wobei das Molekül für die Erkennung von Zielzellen, das Lipidmolekül, welches zur Verbesserung der Zellpenetration befähigt sind, oder das fluoreszierende Markermolekül an die Azidgruppe N₃ von a2 und/oder a2' gebunden wird.

10. Verfahren nach Anspruch 9, wobei die chemische Struktur des Moleküls für die Erkennung von Zielzellen, des Lipidmoleküls, welches zur Verbesserung der Zellpenetration befähigt ist, oder des fluoreszierenden Moleküls gleich 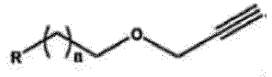 ist, wobei R für eines oder mehrere Elemente von einer Gruppe für die Erkennung von Zielzellen, einer Lipidgruppe, welche zur Verbesserung der Zellpenetration befähigt ist, und einer fluoreszierenden Markergruppe steht, und wobei die Alkinylgruppe an die Azidgruppe N₃ von a2 und/oder a2' gebunden ist, sodass 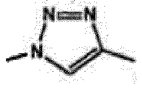 gebildet wird.

11. Verfahren nach Anspruch 7, wobei das Verfahren Folgendes umfasst:
Immobilisieren von a4 beziehungsweise a4', Synthetisieren von X₁ ausgehend von a4 vom 3'- zum 5'-Ende und Synthetisieren von X₂ ausgehend von a4' vom 3'- zum 5'-Ende, sodass a4-X₁ und a4'-X₂ erhalten werden; Anfügen von a5 und a5' an das 5'-Ende von a4-X₁ beziehungsweise a4'-X₂, um a5-X₁-a4 und a5'-X₂-a4' zu bilden;
zunächst Verbinden von a6 mit einem Strang von a5-X₁-a4 und a5'-X₂-a4', Hybridisieren der beiden Stränge, und dann Verbinden von a6 mit dem anderen Strang, sodass sich das komplexe Molekül bildet, wobei a4, a4', a5 und a5' einer der folgenden Gruppen entsprechen: 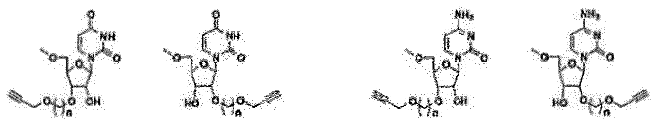 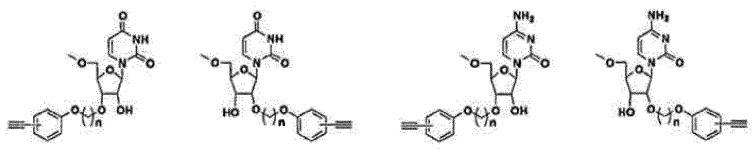 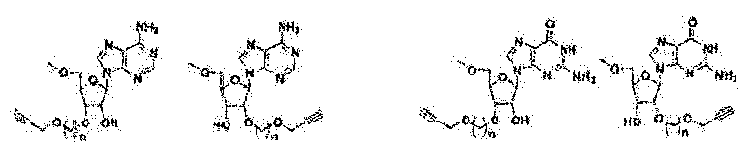 wobei n ist eine ganze Zahl zwischen 0~10 ist;
wobei a6 gleich N₃-(CH₂)p-N₃ ist, wobei p eine ganze Zahl zwischen 2~12 ist.

12. Verwendung des komplexen Moleküls nach Anspruch 1 zur Verbesserung der chemischen Stabilität von siRNA.

13. Verwendung des komplexen Moleküls nach Anspruch 1 zur Verbesserung der Zeitdauer, während derer siRNA im Blut verbleibt.

## Revendications

1. Molécule complexe interférant avec l'expression de gènes cibles, la molécule complexe contenant deux brins d'ARNsi X₁ et X₂ présentant une complémentarité d'au moins 80 %, l'extrémité 5' de X₁ et l'extrémité 3' de X₂ sont liées par une molécule d'acide non-nucléique L₁, et l'extrémité 5' de X₂ et l'extrémité 3' de X₁ sont liées par une molécule d'acide non-nucléique L₂, dans laquelle la molécule d'acide non-nucléique L₁ et la molécule d'acide non-nucléique L₂ sont indépendamment représentées par la formule 1 : 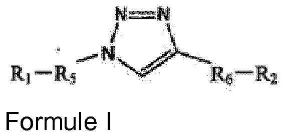 les groupes R₁ et R₂ sont indépendamment un groupe carboxyle, un groupe amino ou un groupe mercapto ;
R₅ est 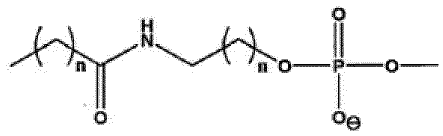 ou 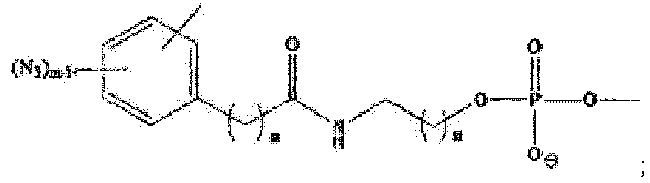 R₆ est l'un des groupes suivants : 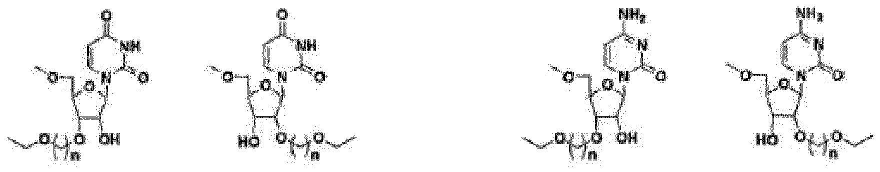 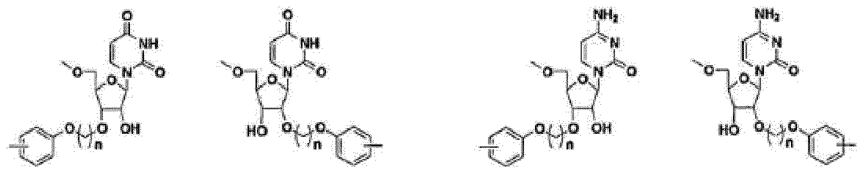 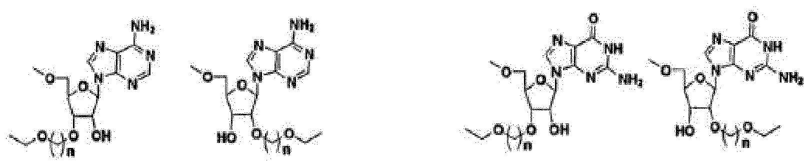 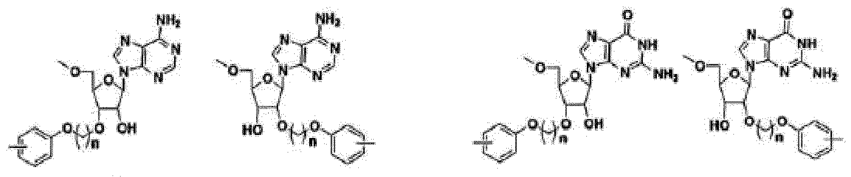 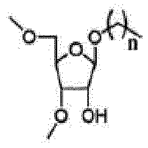 et dans les groupes, n est indépendamment un nombre entier compris entre 0 et environ 10, et m est indépendamment un nombre entier compris entre 1 et environ 5.

2. Molécule complexe selon la revendication 1, dans laquelle la molécule d'acide non-nucléique L₁ est liée de manière covalente à un groupe phosphate ou un groupe hydroxyle de l'extrémité 5' de X₁ et un groupe phosphate ou un groupe hydroxyle de l'extrémité 3' de X₂, et la molécule d'acide non-nucléique L₂ est liée de manière covalente à un groupe phosphate ou un groupe hydroxyle de l'extrémité 5' de X₂ et un groupe phosphate ou un groupe hydroxyle de l'extrémité 3' de X₁.

3. Molécule complexe selon la revendication 1, dans laquelle la molécule d'acide non-nucléique L₁ et/ou L₂ est liée à une ou plusieurs molécules sélectionnées parmi une molécule reconnaissant la cellule cible, une molécule lipidique capable d'améliorer la pénétration cellulaire et une molécule de marquage fluorescente.

4. Molécule complexe selon la revendication 3, dans laquelle
R₅ est 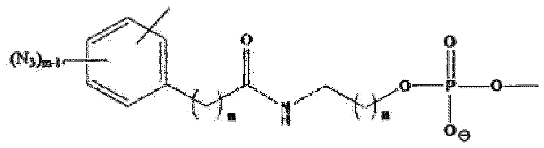 dans les groupes, n est indépendamment un nombre entier compris entre 0 et environ 10, et m est indépendamment un nombre entier compris entre 2 et environ 5,
une ou plusieurs molécules sélectionnées parmi une molécule reconnaissant la cellule cible, une molécule lipidique capable d'améliorer la pénétration cellulaire et une molécule de marquage fluorescente est liée à un groupe azoture N₃ de R₅.

5. Molécule complexe selon la revendication 3 ou la revendication 4, dans laquelle la structure chimique de la molécule reconnaissant la cellule cible, de la molécule lipidique capable d'améliorer la pénétration cellulaire et de la molécule de marquage fluorescente est 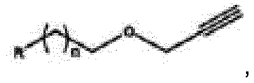 où R est un ou plusieurs groupes sélectionnés parmi un groupe reconnaissant la cellule cible, un groupe lipidique capable d'améliorer la pénétration cellulaire ou un groupe de marquage fluorescent, et le groupe alcynyle dans la structure chimique est lié à L₁ et/ou L₂.

6. Molécule complexe selon la revendication 5, dans laquelle le groupe alcynyle est lié au groupe azoture N₃ de R₅ de L₁ et/ou L₂, de sorte à former

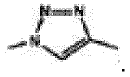

7. Procédé de préparation de la molécule complexe interférant avec l'expression de gènes cibles selon la revendication 1, qui comprend : la préparation de deux brins modifiés d'ARNsi X₁ et X₂ présentant une complémentarité d'au moins 80 %, les deux extrémité 5' et 3' des deux brins d'ARNsi modifiés ayant un groupe liable ; la liaison des groupes liables des extrémités 5' et 3' de X₁ aux groupes liables des extrémités 3' et 5' de X₂ respectivement, de sorte que l'extrémité 5' de X₁ et l'extrémité 3' de X₂ soient liées par une molécule d'acide non-nucléique L₁, et l'extrémité 5' de X₂ et l'extrémité 3' de X₁ sont liées par une molécule d'acide non-nucléique L₂.

8. Procédé selon la revendication 7, lequel procédé comprend : la fixation de a3 et a3' respectivement, la synthèse de X₁ sur a3 à partir de l'extrémité 3' vers l'extrémité 5', et la synthèse de X₂ sur a3' à partir de l'extrémité 3' vers l'extrémité 5', de sorte à obtenir a3-X₁ et a3'-X₂; l'introduction de a1 et a1' sur l'extrémité 5' de a3-X₁ et a3'-X₂ respectivement pour former a1-X₁-a3 et a1'-X₂-a3' ; l'introduction de a2 et a2' sur a1-X₁-a3 et a1'-X₂-a3' respectivement, pour former a2-a1-X₁-a3 et a2'-a1'-X₂-a3' ; et ensuite le recuit de a2-a1-X₁-a3 et a2'-a1'-X₂-a3' ; la soumission de a2 de a2-a1-X₁-a3 et a3' de a2'-a1'-X₂-a3' à une réaction de liaison et la soumission de a3 de a2-a1-X₁-a3 et a2' de a2'-a1'-X₂-a3' à une réaction de liaison, de sorte à former la molécule complexe, où
a1 et a1' sont indépendamment 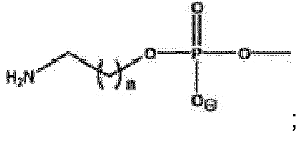 a2 et a2' sont indépendamment 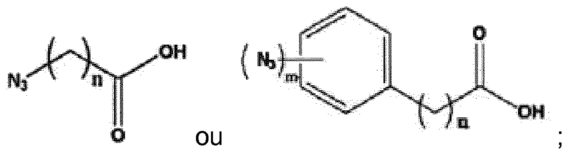 a2-a1 et a2'-a1' sont indépendamment 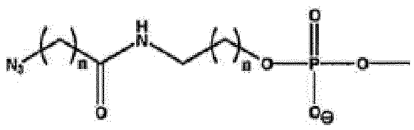 ou 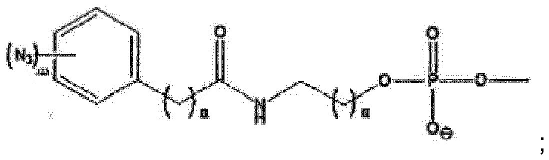 a3 et a3' sont indépendamment un élément des groupes suivants : 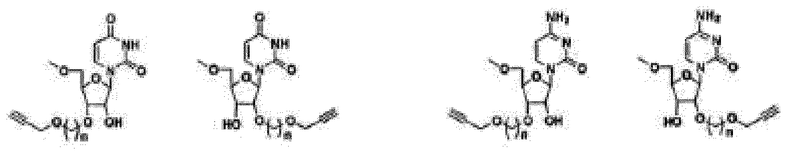 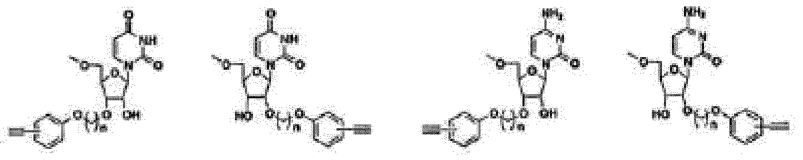 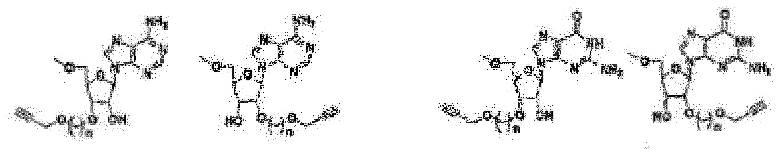 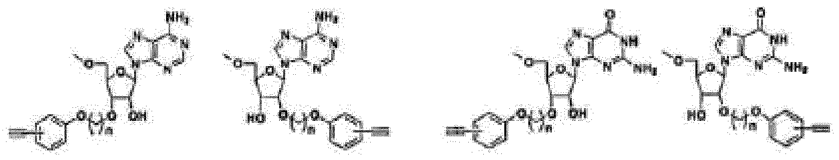 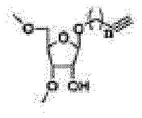 et dans les groupes, n est indépendamment un nombre entier compris entre 0 et environ 10, et m est indépendamment un nombre entier compris entre 1 et environ 5.

9. Procédé selon la revendication 8, dans lequel a2 et/ou a2' sont 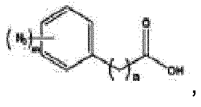 où m est indépendamment un nombre entier compris entre 2 et environ 5 et n est indépendamment un nombre entier compris entre 0 et environ 10, et le procédé comprend en outre la liaison a2 et/ou a2' à une ou plusieurs molécules sélectionnées parmi une molécule reconnaissant la cellule cible, une molécule lipidique capable d'améliorer la pénétration cellulaire et une molécule de marquage fluorescente après la liaison de a2 de a2-a1-X₁-a3 et a3' de a2'-a1'-X₂-a3' et la liaison de a3 de a2-a1-X₁-a3 et a2' de a2'-a1'-X₂-a3', dans lequel la molécule reconnaissant la cellule cible, la molécule lipidique capable d'améliorer la pénétration cellulaire et la molécule de marquage fluorescente est liée au groupe azoture N₃ de a2 et/ou a2'.

10. Procédé selon la revendication 9, dans lequel la structure chimique de ladite molécule reconnaissant la cellule cible, molécule lipidique capable d'améliorer la pénétration cellulaire ou molécule de marquage
fluorescente est 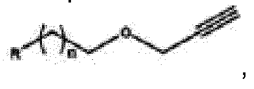 où R est un ou plusieurs groupes sélectionnés parmi un groupe reconnaissant la cellule cible, un groupe lipidique capable d'améliorer la pénétration cellulaire ou un groupe de marquage fluorescent, et le groupe alcynyle est lié au
groupe azoture N₃ de a2 et/ou a2', de sorte à former

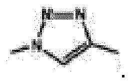

11. Procédé selon la revendication 7, lequel procédé comprend : la fixation de a4 et a4' respectivement, la synthèse de X₁ sur a4 à partir de l'extrémité 3' vers l'extrémité 5', et la synthèse de X₂ sur a4' à partir de l'extrémité 3' vers l'extrémité 5', de sorte à obtenir a4-X₁ et a4'-X₂ ; l'introduction de a5 et a5' sur l'extrémité 5' de a4-X₁ et a4'-X₂ respectivement pour former a5-X₁-a4 et a5'-X₂-a4' ; la liaison tout d'abord de a6 à un brin de a5-X₁-a4 et a5'-X₂-a4', le recuit des deux brins, et ensuite la liaison de a6 à l'autre brin, de sorte à former la molécule complexe, où a4, a4', a5 et a5' sont l'un des groupes suivants : 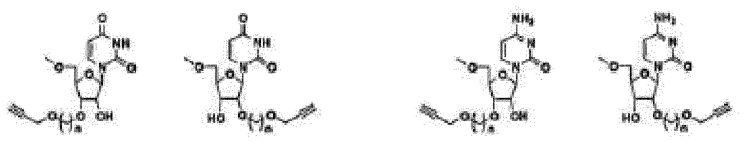 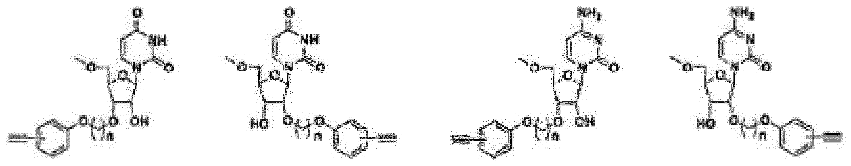 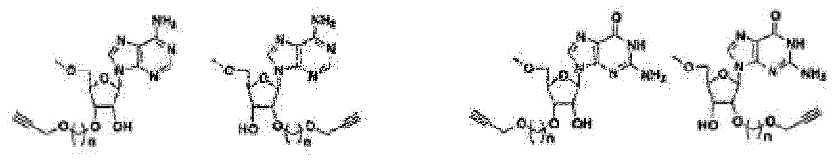 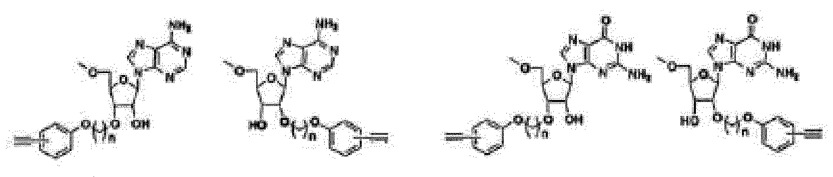 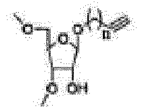 dans lesquels n est un nombre entier comprend entre 0 et environ 10 ;
a6 est N₃-(CH₂)ₚ-N₃, où p est un nombre entier compris entre 2 et environ 12.

12. Utilisation de la molécule complexe selon la revendication 1, pour améliorer la stabilité chimique de l'ARNsi.

13. Utilisation de la molécule complexe selon la revendication 1, pour améliorer le temps de rétention dans le sang de l'ARNsi.
